(19) Europäisches Patentamt / European Patent Office / Office européen des brevets

(11) **EP 3 268 341 B1**

(12) **EUROPEAN PATENT SPECIFICATION**

(45) Date of publication and mention
of the grant of the patent:
**21.04.2021 Bulletin 2021/16**

(21) Application number: **16765474.8**

(22) Date of filing: **11.03.2016**

(51) Int Cl.:
*C07C 51/145* (2006.01)   *C07C 51/21* (2006.01)
*C07C 51/255* (2006.01)   *C07C 63/26* (2006.01)
*B01J 8/08* (2006.01)     *B01J 8/22* (2006.01)

(86) International application number:
**PCT/US2016/021912**

(87) International publication number:
**WO 2016/149060 (22.09.2016 Gazette 2016/38)**

(54) **BUBBLE COLUMN REACTOR BASED DIGESTER AND METHOD FOR ITS USE**

FAULBEHÄLTER AUF BASIS EINES BLASENSÄULENREAKTORS UND VERFAHREN ZU DESSEN VERWENDUNG

DIGESTEUR À BASE DE RÉACTEUR COLONNE À BULLES ET SON PROCÉDÉ D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.03.2015 US 201514657523**

(43) Date of publication of application:
**17.01.2018 Bulletin 2018/03**

(73) Proprietor: **GRUPO PETROTEMEX, S.A. DE C.V.**
**San Pedro Garza Garcia, Nuevo Leon, 66265 (MX)**

(72) Inventor: **WONDERS, Alan, G.**
**Richardson, TX 75080 (US)**

(74) Representative: **Tostmann, Holger Carl**
**Wallinger Ricker Schlotter Tostmann**
**Patent- und Rechtsanwälte Partnerschaft mbB**
**Zweibrückenstrasse 5-7**
**80331 München (DE)**

(56) References cited:
**US-A1- 2006 205 976     US-A1- 2007 155 985**
**US-A1- 2007 293 699     US-B2- 7 816 556**

EP 3 268 341 B1

## Description

FIELD OF THE INVENTION

This invention relates to a method to oxidatively digest crude terephthalic acid particles to obtain purified terephthalic acid particles of useful purity and particle size distribution that is economically efficient, has reduced energy costs, equipment capital costs, maintenance and reliability costs, and has minimal or no over-oxidation of organic solvent and aromatic compounds to carbon oxides (CO and $CO_2$) and to the apparatus for conducting the method.

BACKGROUND OF THE INVENTION

Terephthalic acid is a commercially important feedstock for a variety of applications, primary of which is the production of polyethylene terephthalate (PET). PET is a well-known plastic used in great quantities around the world to make products such as bottles, fibers, and packaging.

Terephthalic acid is conventionally produced by liquid-phase oxidation of para-xylene. In a typical liquid-phase oxidation process, a liquid-phase feed stream and a gas-phase oxidant stream are introduced into a primary oxidation reactor and form a multi-phase reaction medium in the reactor. The liquid-phase feed stream introduced into the primary reactor contains para-xylene, while the gas-phase oxidant stream contains molecular oxygen. At least a portion of the molecular oxygen introduced into the primary reactor as a gas dissolves into the liquid phase of the reaction medium to provide oxygen availability for the liquid-phase reaction. If some portions of the liquid phase of the multi-phase reaction medium contain an insufficient concentration of molecular oxygen (i.e., if certain portions of the reaction medium are "oxygen-starved"), undesirable side-reactions can generate impurities and/or the intended reactions can be retarded in rate. If the liquid phase of the reaction medium contains too little of the oxidizable compound, the rate of reaction may be undesirably slow relative to the over-oxidation reactions. Further, if the liquid phase of the reaction medium contains an excess concentration of the oxidizable compound, additional undesirable side-reactions can generate impurities.

The solvent present in the liquid-phase generally comprises a low molecular weight organic acid such as acetic acid and water. In production systems wherein the solvent is recycled, the solvent may contain small quantities of impurities such as, for example, para-tolualdehyde, terephthaldehyde, 4-carboxybenzaldehyde (4-CBA), benzoic acid, para-toluic acid, para-toluic aldehyde (4-methylbenzaldehyde), alpha-bromo-para-toluic acid, isophthalic acid, phthalic acid, trimellitic acid, polyaromatics, and/or suspended particulate.

The catalyst system conventionally employed in the partial oxidation of para-xylene is a homogeneous, liquid-phase catalyst comprising cobalt, bromine, and manganese.

The use of bubble column reactors for the primary oxidation reaction offers many advantages over conventional continuous stirred tank reactors, and oxidation processes employing bubble column reactors are disclosed, for example, in U.S. 7,355,068, U.S. 7,371,894, U.S. 7,568,361, U.S. 7,829,037, U.S. 7,910,769, U.S. 8,501,986, U.S. 8,685,334 and U.S. 8,790,601. Bubble column reactors provide agitation of the reaction medium without requiring expensive and unreliable mechanical equipment. Bubble column reactors typically include an elongated upright reaction zone within which the reaction medium is contained. Agitation of the reaction medium in the reaction zone is provided primarily by the natural buoyancy of gas bubbles rising through the liquid phase of the reaction medium. This natural-buoyancy agitation provided in bubble column reactors reduces utility power, capital, and maintenance costs relative to mechanically agitated reactors. Further, the substantial absence of moving mechanical parts associated with bubble column reactors provides an oxidation system that is less prone to mechanical failure in comparison to mechanically agitated reactors.

In liquid-phase partial oxidation of para-xylene the product withdrawn from the primary oxidation reactor is typically a slurry comprising a particulate solid-phase of crude terephthalic acid (CTA) and a mother liquor. CTA contains relatively high levels of impurities (e.g., 4-carboxybenzaldehyde, para-toluic acid, fluorenones, and other color bodies) that render it unsuitable as a feedstock for the production of PET. Thus, the CTA is typically subjected to a purification process that converts the CTA particles into purified terephthalic acid (PTA) particles that may be suitable for production of polyethylene terephthalate. In recent commercial practice, the further purification of CTA is often by a hydrogenation process or an oxidative digestion process.

A conventional hydrogenation process for converting CTA to PTA may include the following steps: (1) replacing the mother liquor of the CTA-containing slurry with water, (2) heating the CTA/water slurry to dissolve the CTA in water, (3) catalytically hydrogenating the CTA/water solution to convert impurities to more desirable and/or easily-separable compounds, (4) selectively precipitating the terephthalic acid from the hydrogenated solution via multiple crystallization steps, and (5) separating the crystallized PTA from the remaining liquids. Although effective, this type of conventional purification process can be very expensive. Individual factors contributing to the high cost of conventional CTA purification methods include, for example, the heat energy required to promote dissolution of the CTA in water, the catalyst required for hydrogenation, the hydrogen stream required for hydrogenation, the yield loss caused by hydrogenation of some terephthalic acid, and the multiple vessels required for multi-step crystallization.

Alternatively, CTA may be converted to PTA in a series of additional oxidation reactors commonly referred to as "digesters." Typically in such a system an initial slurry of CTA particles in an initial oxidation reaction mother liquor may contain from 10 to 50 weight percent solid CTA particles, with the balance being liquid mother liquor. The solid CTA particles present in the initial slurry withdrawn from primary oxidation reactor may contain from 400 ppmw to 15,000 ppmw of 4-carboxybenzaldehyde (4-CBA). The initial oxidation reactor system may include both a primary oxidation reactor providing principally for oxidizing the majority of the liquid phase oxidizable compound and optionally at least one secondary oxidation reactor providing principally for polishing conversion of liquid phase oxidizable compound prior to entering the digesters.

Typically the CTA slurry withdrawn from the initial oxidation reactor system is transferred to a system of digester units wherein further oxidation reaction is conducted at slightly to significantly higher temperatures than were used in the primary and optional secondary oxidation reactors. Optionally, the slurry of CTA particles may be subjected to a solvent replacement step before proceeding to the digester units, whereby the replaced solvent has reduced concentrations of aromatic impurities and/or altered concentrations of catalyst and water that are readjusted to be more suitable for oxidation catalysis in the digester units. Optionally, the mass fraction of solids in the CTA slurry may also be adjusted, with or without solvent replacement, prior to entering the digester units.

The CTA particles obtained by relatively rapid and heterogeneous precipitation in the primary oxidation reactor typically have a relatively large degree of crystalline imperfections including high porosity, high surface area, and small and non-uniform particle size, and the dried CTA typically exhibits a lower bulk density than CTA obtained in CSTR oxidations. In addition, the relatively rapidly precipitated CTA particles typically comprise super-equilibrium concentrations of many of the oxidation impurities, including coupled polyaromatic species and incomplete oxidation intermediates. The incomplete oxidation intermediates, such as 4-CBA and para-toluic acid are particularly troublesome since they typically occur at concentrations of a few hundred to several thousand parts per million by weight. In contrast, usage of the PTA in forming condensation polymers such as PET require concentrations of mono-carboxylic acid polymer chain terminators as low as possible, with 300 parts per million being a typical upper limit for the sum of all mono-carboxylic acids in PTA .

More preferably, the CTA slurry from primary oxidation is treated in a secondary oxidation BCR before being fed to digestion. The principal objective of this secondary oxidation, which is also referred to as Post Oxidation or as Early Oxidative Digestion as in U.S. 7,393,973, is to oxidize a substantial fraction of the liquid phase aromatic oxidation intermediates from primary oxidation onwards to TPA before entering the more severe oxidation conditions of digestion. This provides a useful reduction in the total amount of over-oxidation to carbon oxides incurred subsequent to primary oxidation.

In order to make the precipitated oxidation intermediate impurities available for oxidation in the series of digesters, the particles are exposed to higher temperatures than in the primary oxidation to at least partially dissolve the CTA particles and expose the impurities to liquid-phase oxidation comprising additional molecular oxygen injected into the digester. The high surface area, crystalline imperfections, and super-equilibrium impurity concentrations of the small CTA particles are favorable, both kinetically and thermodynamically, for partial dissolution and on-going recrystallization of the terephthalic acid when the CTA slurry temperature is raised moderately above the temperature at which the CTA was formed in the primary oxidation.

The further oxidation conducted in the digester system is intended to reduce the concentration of 4-CBA in the CTA particles. The digestion temperature may be from 5°C to 90°C higher than the primary oxidation temperature and typically may be from about 150°C to 280°C. The purified product from the oxidative digestion may be crystallized and collected in one or more crystallization and recrystallization units.

In a second effect of the digestion process, the terephthalic acid particles may experience Ostwald ripening which tends to provide larger particles having a narrowed particle size distribution in comparison to the CTA particles in the outlet stream of the primary oxidation.

In a third effect of the digestion process, the recrystallized terephthalic acid particles comprise reduced concentrations of many of the impurities that are resistant to catalytic oxidative correction to form terephthalic acid, impurities such as polyaromatic carboxylic acid species, notably including many colored species such as 2,6-DCF and 2,7-DCF, inter alia. This reduction is caused by a closer approach to equilibrium distribution of the oxidation resistant impurities between solid and liquid phases, resulting from both the hotter operating temperature than in initial oxidation and also to the extended recrystallization time during the digestion process. The reduction in solid phase concentration of oxidation resistant impurities is further enhanced if the optional solvent replacement step has used a relatively more pure solvent such as, for example, distilled aqueous acetic acid from a solvent dehydration process used for removing the water produced by oxidation of the para-xylene.

Bubble column digesters (BCR) provide the mechanical advantages described above for bubble column oxidation reactors. However, when conventional multiple BCRs in series or in parallel are employed, this leads to large production plant footprint as well as intricate plumbing systems to supply and control the multiple columns. In addition, the design of a CTA digestion process and BCR apparatus is, however, presented with a number of design difficulties different than initial oxidations BCRs while arriving at the beneficial process objectives for digestion.

Several of these digestion process design objectives are addressed in U.S. 7,393,973. Specifically of note for the present invention, preferred ranges of residence time distribution (RTD) for the liquid and solid phases that are useful in providing the competing process objectives near the solids inlet and near solids outlet, a means of reducing the short-circuiting of feed solids from inlet to solids outlet, and a means of controlling over-oxidation to carbon oxides while nonetheless achieving the desired oxidation of partial oxidation intermediates onwards to product TPA.

As disclosed in U.S. 7,393,973, the solids dissolution rate is particularly rapid near the solids entry to digestion owing to the local concentration of a greater fraction of smaller particles obtained from initial oxidation under the disclosed bubble column conditions. Accordingly, near the solids inlet in contrast to near the solids outlet, there is a local need for a greatest supply rate of dissolved oxygen from the gas phase into the liquid phase owing to the faster rate of dissolving oxidization intermediates, e.g., 4-CBA and para-toluic acid, from the solid phase. A closely related difficulty near the solids inlet is the need for an apt combination of liquid mixing dilution and concurrent oxidation such that the standing concentration of liquid phase oxidation intermediates, e.g., 4-CBA and para-toluic acid, are kept sufficiently small to prevent too much reprecipitation into the larger, more thermodynamically and kinetically stable particles of PTA that are being formed. In combination, these difficulties recommend a greater degree for local slurry mixing and/or aeration at the solids inlet region of the digester in contrast to the solids outlet region in order to dilute the burst of dissolving aromatic oxidation intermediates and to provide the necessary local supply rate of dissolved molecular oxygen. The local supply rate for dissolved oxygen can be provided by various combinations of greater mole fraction of gaseous molecular oxygen and greater aeration for improved interphase mass transfer coefficient, commonly denoted as the product "kLa" of a interphase film transfer effect "kL" and an interfacial area property "a".

Another digestion process difficulty disclosed in U.S. 7,393,973 is the need to limit the short-circuit passage of solids fed from initial oxidation from their entry location into digestion to the solids outlet from digestion. Short-circuiting particles will carry with them a greater than desired concentration of solid-phase oxidation intermediates thereby undesirably skewing upwards the weighted average composition of oxidation intermediates, e.g., 4-CBA, in all exiting solids. In addition, short-circuiting particles will also undesirably broaden the particle size distribution and reduce the variously computed average particle sizes for the solid PTA exiting the digestion process; and this adversely affects in varying degrees the filtration, washing, drying, and bulk handling of PTA powder product.

Pertaining to the difficulty for balancing in digesters between the desirable oxidation of aromatic oxidation intermediates against over-oxidation of carboxylic acids, e.g., acetic acid and TPA, to forming carbon oxides, the disclosure of U.S. 7,393,973 describes that "the later stage of oxidative digestion is carried out under "oxygen-starved" conditions, where a very low concentration of molecular oxygen is present in the gaseous effluent". This is also combined with a temperature differential wherein the temperature of the later stage is greater than the earlier stage. Further according to this invention, "...molecular oxygen is supplied to digestion at multiple elevations ...", and "... the separated elevations for supplying molecular oxygen to digestion comprise at least one opening in the upper half of the digestion reaction medium and at least one opening in the lower half of the digestion reaction medium".

Multiple digester unit configurations are described in U.S. 7,393,973. However, a digester system based solely on bubble column technology is not described. As noted earlier herein, bubble column reactors (BCRs) offer mechanical and cost efficiencies in comparison to mechanically agitated reactors which have high capital cost, high operating cost, and high operational and maintenance requirements.

CN 202700501 describes a TPA process wherein "deep-oxidating" (digestion) is conducted in one or more BCRs. After primary oxidation in a bubble column oxidation reactor, the oxidation reaction slurry is conducted through a smaller diameter "discharge barrel" wherein the primary oxidation process is continued. The primary oxidation slurry is transferred from the discharge barrel into a middle portion of a first bubble column "deep oxidating reactor" which is fed with an oxygen containing gas at the bottom of the BCR. In one embodiment, only one deep oxidating reactor is employed. In a second embodiment, the slurry taken from the bottom of the first deep oxidating reactor is conducted into a middle portion of a second deep oxidating reactor also having an air inlet at the bottom. The exhaust gas of the first deep oxidating reactor is piped to near the bottom of the second deep oxidating reactor and is the source of the oxygen containing gas for the second deep-oxidating reactor. In both embodiments, off-gas from the primary oxidating reactor is used to dilute ambient air before compression and feeding to the first deep-oxidating reactor. Although a specific volume ratio for the deep-oxidating BCRs of 70-80% in comparison to the "oxidating" BCR (primary oxidizer) along with a length-to-diameter ratio in the range of 5 to 8 is described, there is no disclosure for primary oxidizer or the deep-oxidating BCR vessels re their volumes with respect to liquid and solid flow rates. Furthermore, there is no disclosure of an apt ratio of the flow of molecular oxygen, mole fraction of molecular oxygen, or flow of total gas compounds comprised in the combination of off-gas recycle plus ambient air in comparison to either CTA slurry flows or vessel diameters in the deep-oxidating BCRs. These gas flow rates and molecular oxygen fractions provided to the deep-oxidating reactors are essential to controlling the energy requirements, the mixing and mass transfer hydrodynamics, the desirable chemical reactions comprising liquid phase oxidation reactions of para-toluic acid and 4-CBA to form terephthalic acid, the carbon burning reactions wasting carboxylic acids to CO and CO2, the sedimentation of growing solid particles in the absence of mechanical agitation, and the residence time distribution of the solids within the deep-oxidating BCRs. In summary, CN 202700501 provides insufficient disclosure of features essential to scaling and design of 3-phase BCRs for efficacious digestion conversion of CTA to PTA.

US 7,816,556 B2 discloses a system for the producing of terephthalic acid. The system includes a multi-stage oxidative digestion system for the processing of an improved initial slurry from primary oxidation. The multi-stage digestion system is capable of adequately purifying the improved initial slurry without requiring significant liquor exchange between the primary oxidation and the oxidative digestion.

Thus there is a need for a digestion method and a correspondingly structured reactor for CTA slurries obtained in an initial oxidation of para-xylene in bubble column units that is efficient, reduced in cost in requirement for capital installation, mechanical maintenance, process utilities, raw material losses and simple in design to conduct the digestive oxidation and produce good quality PTA.

SUMMARY OF THE INVENTION

Therefore, it is an object of the present invention to provide a method for effective and efficient digestion of CTA particles that provides terephthalic acid at a quality level necessary for the production of PET.

It is also an object of the present invention to provide a digestion system structure wherein CTA is converted to terephthalic acid at a quality level necessary for the production of PET.

These and other objects are solved by a method for purification of crude terephthalic acid according to claim 1, an oxidative digestion system according to claim 3, and a bubble column digestion system according to claim 12.

The inventive method for purification of crude terephthalic acid is as defined in claim 1.

In a second embodiment the present invention includes an oxidative digestion system as defined in claim 3. In a third embodiment, the present invention provides a bubble column digestion system as defined in claim 12.

The oxidative digestion systems according to the above embodiments are free of mechanical agitation.

The forgoing description is intended to provide a general introduction and summary of the present invention and is not intended to be limiting in its disclosure unless otherwise explicitly stated. The presently preferred embodiments, together with further advantages, will be best understood by reference to the following detailed description taken in conjunction with the accompanying drawings.

BRIEF DESCRIPTION OF THE DRAWINGS

Fig. 1 is a schematic drawing of a para-xylene oxidation system including a digester oxidation unit according to one embodiment of the present invention.

Fig. 2 is a schematic drawing of a digester oxidation unit according to an optional embodiment wherein two separate BCRs are used in series flow on the slurry phase with a fresh feed supply of molecular oxygen to each BCR.

Fig. 3 is a schematic drawing of a digester oxidation unit according to an embodiment of the invention having lower zones of lesser diameter than the uppermost zone.

Fig. 4A is a schematic diagram of a baffle arrangement from a horizontal perspective according to one embodiment of the present invention.

Fig. 4B is a schematic diagram of one baffle of 4A from a vertical perspective.

Fig. 5A is a schematic diagram of another baffle from a horizontal perspective according to one embodiment of the present invention.

Fig. 5B is a schematic diagram of the baffle of 5A from a vertical perspective.

Fig. 6 is a schematic drawing of an inverted cone deflector unit for a slurry feed inlet according to an embodiment of the present invention.

DESCRIPTION OF THE PREFERRED EMBODIMENTS

In the description of the present invention the terms "a," "an," and "the" mean one or more. The term "and/or," when used in a list of two or more items, means that any one of the listed items can be employed by itself or any combination of two or more of the listed items can be employed. For example, if a composition is described as containing components A, B, and/or C, the composition can contain A alone; B alone; C alone; A and B in combination; A and C in combination, B and C in combination; or A, B, and C in combination. The terms "comprising," "comprises," and "comprise" are open-ended transition terms used to transition from a subject recited before the term to one or more elements recited after the term, where the element or elements listed after the transition term are not necessarily the only elements that make up the subject. The terms "having," "has," and "have" as well as the terms "including," "includes," and "include" have the same open-ended meaning as "comprising," "comprises," and "comprise" provided above.

As used herein, the gas hold-up, bubble hold-up, gas fraction, and bubble fraction of an aerated reaction medium all mean the same and are simply the volume fraction of a multi-phase medium that is in the gaseous state. The units are dimensionless, being cubic meters divided by cubic meters, for example, or they can be stated as volume percent. As used herein, the superficial gas velocity is the volumetric flow rate of components of a multi-phase medium that are in the gaseous state divided by the cross sectional area of the conduit or vessel through which these gaseous components are flowing. The units of superficial gas velocity are cubic meters per second divided by square meters, resulting in meters per second, for example. Superficial slurry velocity, superficial liquid velocity, and superficial solid velocity are similarly defined replacing gaseous component volumetric flow rate with slurry volumetric flow rate, liquid volumetric flow rate and solid volumetric flow rate, respectively. It is important to observe that the gas superficial velocity is a computed value and does not necessarily represent the actual spatial velocity of any bubble or other aliquot of gaseous component. Similar statements apply to the other superficial velocities. Is important to observe that the liquid and solid superficial velocities may be different from each other and in turn different from the slurry superficial velocity when the effects of liquid-solid density difference and gravitational force are relatively greater compared to the prevailing local convective liquid turbulence, i.e. when there is significant solids sedimentation owing to reduced liquid phase turbulence and/or due to increased particles sizes of the solid phase. As used herein air means any gaseous mixture comprising molecular oxygen in any mole fraction, unless further explicit modification is provided such as ambient air, compressed ambient air, and ambient air plus recycled off-gas, inter alia.

Where numerical ranges are provided to quantify certain parameters relating to the invention it should be understood that such ranges are to be construed as providing literal support for claim limitations that only recite the lesser value of the range as well as claim limitations that only recite the greater value of the range. The values expressed also include ranges and sub-ranges within the stated limit values.

According to the present invention, a slurry of crude terephthalic acid (CTA) obtained via a primary oxidation operation such as described in U.S. 7,355,068, U.S. 7,393,973, U.S. 7,829,037, U.S. 7,910,769, U.S. 8,501,986, U.S. 8,685,334 and U.S. 8,790,601 is treated in a column structure digestion unit comprising a series of vertically arranged zones. The CTA slurry contains particles of crude terephthalic acid, comprising terephthalic acid, 4-carboxybenzaldehyde, p-toluic acid in an acetic acid medium as described previously. For digestion, the temperature of the CTA slurry obtained from the primary oxidation system is increased to a value of at least 5°C to 90°C higher, preferably from 10°C to 60°C higher, and more preferably from 15°C to 40°C higher than the temperature of slurry entering from initial oxidation. The temperature of slurry leaving initial oxidation may be in the range of from 125°C to 200°C, preferably from 140°C to 185°C, more preferably from 150°C to 175°C. Thus, the temperature of the slurry in a digestion stage is in the range of from 150°C to 280°C, preferably from 160°C to 240° C, and more preferably from 170°C to 210°C. This increase in temperature may be accomplished in a heating unit located prior to the digestion unit or by any method suitable for increasing the temperature of a slurry of particles in an aqueous acetic acid mixture.

The increased temperature in digestion typically comprises a greater overhead pressure than in primary and secondary oxidation in order to control evaporation of solvent in the digestion zones. The overhead off-gas pressure of at least one digestion zone relative to the overhead off-gas pressure of the primary oxidation is in the range of from about the same as to 4MPa greater, preferably from 0.5 MPa greater to 3 MPa greater, and most preferably from 1 MPa greater to 2 MPa greater. Preferably, the overhead off-gas pressure of at least one digestion zone is in the range of from 0.4 MPa to 8 MPa, from 0.5 MPa to 4 MPa, or from 1 MPa to 2 MPa.

The present inventors have discovered that it is possible to feed slurry from a position of lesser gauge pressure at an upper elevation in the primary oxidizer BCR into a position of greater gauge pressure located at a lower elevation in the secondary oxidizer BCR without using a mechanical pumping means or any supplementary flow energy, such as an eductor, and despite the typically greater density of reaction medium in the secondary oxidizer BCR. Furthermore, the inventors have discovered that feeding CTA slurry from a primary oxidizer to a lower elevation of the secondary oxidizer BCR is surprisingly beneficial for optimizing the objectives of increased conversion of liquid phase aromatic intermediates with reduced formation of coupled and colored polyaromatic compounds simultaneously with reduced over-oxidation reactions forming carbon oxides.

The transfer from lesser gauge pressure to higher gauge pressure is preferably accomplished by segregating aerated reaction medium drawn from an upper elevation of a primary oxidizer and deaerating it to form a substantially de-aerated slurry. More preferably the deaeration of reaction medium is accomplished in a minimizing amount of time according to the disclosures of U.S. 7,371,894 thereby minimizing the formation of aromatic impurities comprising polyaromatic and colored impurities. Either an internal or external deaeration vessel is preferred. More preferably, the deaeration vessel for the reaction medium is external to the primary oxidizer.

The deaerated slurry drawn from a lower portion of the deaeration vessel has a greater bulk density and elevation head per meter of height than the aerated slurry in either the primary or secondary oxidizer BCRs. Accordingly a connecting conduit from a lower portion of the deaeration vessel can flow by gravitational force alone into any lower elevation of either the primary or secondary oxidizer BCRs when they are operating in an aerated condition. Essentially, the gas and solvent vapors rising upwards in the primary oxidizer BCR have expanded the reaction medium in a gas-lift type pumping, although the slurry retained within the primary oxidizer BCR is continually falling back downwards through the rising gas according to the force of gravity interacting with viscous and surface tension forces, among others.

However, as a practical matter of process control, it is useful and preferred to have certain relationships among the elevation of the base of the deaeration vessel exiting the primary oxidizer, the elevation of the top of the aerated reaction medium in the secondary oxidizer, the elevation at which aeration is first provided within the secondary oxidizer BCR, and the degree of aeration within the secondary oxidizer BCR. This is to ensure that sufficient slurry flow can be conveyed with conduits and flow control elements of commercially reasonable size.

It is preferred that the gas hold-up in the secondary oxidizer BCR be at least 14, more preferably at least 20, still more preferably at least 26, most preferably at least 32 volume percent at normal operating conditions of various flows, pressures, temperatures and levels within the primary and secondary BCRs. Besides considerations for supporting sufficient flow and control of slurry drawn from the upper elevation of the primary oxidizer BCR, this degree of aeration is useful for supporting mass transfer from gas-to-liquid for supplying dissolved molecular oxygen in the secondary oxidizer BCR. It is preferred to achieve this gas hold-up using superficial gas velocities of at least 0.04, more preferably at least 0.08, still more preferably at least 0.12, most preferably at least 0.16 meters per second measured or calculated at the BCR mid-height elevation, quarter-height elevation, and three-quarter height elevation. This superficial velocity is calculated using the volumetric flow of all rising gaseous components, both true gases, e.g. $O_2$, $N_2$, Ar, CO, $CO_2$ and the like, and evaporated liquid vapors, e.g. acetic acid vapor, water vapor, and the like, divided by the cross sectional area of the BCR at the considered elevation.

It is preferred that at least 25, more preferably at least 50, still more preferably at least 75, most preferably at least 100 percent of the slurry fed to the secondary oxidizer BCR be withdrawn from the primary oxidizer BCR at an elevation that is at least 12, more preferably at least 18, still more preferably at least 24, most preferably 30 meters above the lowest elevation at which gaseous molecular oxygen is supplied to the primary oxidizer BCR.

It is preferred that at least 25, more preferably at least 50, still more preferably at least 75, most preferably at least 100 percent by mass of the slurry withdrawn from the upper elevation of the primary oxidizer BCR be supplied to the secondary oxidizer BCR at an elevation that is less than 12, more preferably less than 8, still more preferably less than 4, most preferably less than 2 meters above the lowest elevation at which gaseous molecular oxygen is supplied to the secondary oxidizer BCR.

It is preferred that the normal operating level of the secondary oxidizer BCR is less than 45, more preferably less than 40, still more preferably less than 35, most preferably less than 30 meters and more than 8, more preferably more than 12, still more preferably more than 16, most preferably more than 20 meters above the lowest elevation at which gaseous molecular oxygen is supplied to the secondary oxidizer BCR.

It is preferred that the tangent line to tangent line height of the secondary oxidizer BCR is less than 50, more preferably less than 45, still more preferably less than 40, most preferably less than 35 meters and more than 10, more preferably more than 14, still more preferably more than 18, most preferably more than 22 meters.

It is preferred that the lowest elevation at which gaseous molecular oxygen is supplied to the secondary oxidizer BCR is less than 16, more preferably less than 12, still more preferably less than 8, most preferably less than 4 meters displaced vertically from the lowest elevation at which gaseous molecular oxygen is supplied to the primary oxidizer BCR.

It is preferred to select a combination from the above ranges for normal operating level and normal gas hold-up within the secondary oxidizer BCR such that the gauge pressure in the slurry supply conduit at the highest elevation of slurry entry into the secondary oxidizer from the primary oxidizer is greater than the pressure inside the secondary oxidizer BCR at the same elevation by at least 30, preferably at least 60, still more preferably at least 90, most preferably at least 120 kPa when the slurry flow is quickly stopped, i.e. the pressure differential between the slurry supply conduit and the inside of the secondary oxidizer BCR at the highest elevation of slurry entry measured or calculated without any pressure loss in the supply conduit relating to frictional flow losses, kinetic head pressure losses, or control element pressure losses. The excess of static gauge pressure in the slurry supply conduit compared to the gauge pressure inside the secondary oxidizer BCR, both measured at the highest elevation where slurry is supplied to the secondary oxidizer BCR, is thereby available for kinetic head development, for frictional loses in the slurry conduit and entry to the secondary oxidizer BCR, and for control elements to regulate the flow rate of slurry. The slurry flow rate from the primary oxidizer into the secondary oxidizer is preferably regulated in response to at least one process measurement comprising the level of aerated reaction medium in the primary oxidizer BCR, the level of aerated reaction medium in the secondary oxidizer BCR, the volumetric or mass feeding rate of para-xylene into the primary oxidizer, and the mass or volumetric flow rate of slurry into and/or out of the secondary oxidizer.

The surprising benefits of feeding the slurry from the primary oxidizer BCR to near the base of the secondary oxidizer BCR pertain to matching the region of greatest gas-to-liquid mass transfer supply rate of dissolved molecular oxygen with the region of greatest demand for dissolved molecular oxygen in combination with providing a greater axial separation of slurry entry and exit elevations. For example, when slurry is fed at a middle elevation, perhaps to place the peak demand for dissolved oxygen closer to a fresh supply of gaseous molecular oxygen or perhaps to allow the liquid phase concentration of dissolved aromatic intermediates to bloom both upwards and downwards to reduce the peak demand rate for dissolved molecular oxygen, then the concentration of aromatic intermediates in a BCR bottom outlet is also undesirably increased. By feeding the slurry and the gaseous supply of molecular oxygen both near the base and then withdrawing the slurry from near the top of the secondary oxidizer BCR, it is possible to achieve simultaneously the benefits of reduced formation of coupled polyaromatic impurities, reduced need for an excess of molecular oxygen near the top of the secondary oxidizer BCR, and increased conversion of the liquid phase partial oxidation intermediates in the slurry leaving the secondary oxidizer. The increased conversion of liquid phase partial oxidation intermediates can provide a reduced carbon burning in the subsequent digester operation, particularly when the CTA slurry is fed to directly to the digestion bypassing a solvent exchange operation. Accordingly, it is preferred that at least 25, more preferably at least 50, still more preferably at least 75, most preferably at least about 100% of the supply of gaseous molecular oxygen to the secondary oxidizer BCR be fed within 1, preferably within 0.5, still more preferably within 0.25, most preferably within 0.1 times the diameter of the main cylindrical portion and within 0.64, more preferably within 0.32, still more preferably within 0.16, most preferably within 0.08 meters of the lowest elevation within the secondary oxidizer BCR. Also, it is preferred that at least 25, preferably at least 50, still more preferably within 75, most preferably at least about 100% of the supply of CTA slurry to the secondary oxidizer BCR be fed within 3, more preferably within 2, still more preferably within 1, most preferably within 0.5 times the diameter of the main cylindrical portion and within 6, more preferably within 4, still more preferably within 2, most preferably within 1 meters of the lowest elevation within the secondary oxidizer BCR.

The increased conversion of liquid phase oxidation intermediates in the elevated slurry outlet is obtained because the secondary oxidizer BCR is not a perfectly mixed vessel with respect to the reaction and mass transfer time constants relevant in the partial oxidation of para-xylene to form TPA. Accordingly, when the entry and exit points for slurry can be widely separated, the reaction conversion is improved even without changing the size of the column. It is preferred that the slurry inlets and outlets of the secondary oxidizer BCR be separated vertically by at least 8, more preferably at least 12, still more preferably at least 16, most preferably at least 20 times the maximum diameter of the secondary oxidizer BCR. Additionally, it is preferred that the slurry outlet of the secondary oxidizer BCR be located less than 8, more preferably less than 6, still more preferably less than 4, most preferably less than 2 times the maximum diameter of the main cylindrical portion of the vessel below the normal, time-averaged operating level of the reaction medium. The portion of the vessel volume that is above the time-averaged operating level can be used for gas disengagement and/or level fluctuations relating to responses to upstream and downstream flow rate disturbances. In one preferred embodiment of the invention, the normal, time-averaged operating level of the reaction medium in a secondary oxidizer BCR is located substantially at the slurry outlet elevation, whereby the vessel is operating in a slurry overflow mode.

However, increased axial staging provided by more widely separating slurry entry and exit positions and/or making the secondary oxidizer BCR volume larger can reach a situation of diminishing returns on liquid phase oxidation compared to what remains in the solid phase particles.

To avoid too much conversion of the liquid phase para-toluic acid, the volume of the secondary oxidizer BCR can be reduced to provide a reduction in capital cost and also in over-oxidation to carbon oxides. In this situation, it is often desirable to reduce the vessel inside diameter "D" in preference to the vessel height "H" so that the H:D ratio of the BCR increases and thereby promotes greater staging for the reaction volume that remains. Of course, the volume reduction will increase the concentration of liquid phase oxidation intermediates exiting the secondary oxidizer BCR, but reducing the vessel volume by reducing diameter provides a much attenuated shift compared to reducing volume by reducing height. Accordingly, it is preferable that the height to diameter ratio of the reaction medium in the secondary oxidizer BCR be in the range of at least 6:1, more preferably at least 12:1, still more preferably at least 16:1, most preferably 20:1.

It is also useful to increase the axial staging of the liquid phase oxidation in the secondary oxidizer BCR by using at least 1, 2, 4, 8 "non-fouling" baffles according to the disclosure of U.S. 7,568,361. (It is noted that throughout the remainder of the specification the listing of multiple numerical values as in the preceding sentence signifies a nested range from preferred to most preferred). This becomes increasingly important in production units sized for greater production rates wherein a tall H:D ratio indicates excessively tall absolute heights. Addition of non-fouling baffles can sustain a good staging performance in the secondary oxidizer BCR even with increasing D and declining H:D.

It is generally preferred to place the majority of these baffles above the sedimentation level of solids that is realized after the feeding of fresh molecular oxygen is suspended for at least 2, 8, 16, 32 hours. After the CTA solids are settled and compacted, very large forces can be imposed on a baffling means when aeration is restarted rapidly. Nonetheless, it is possible to provide non-fouling baffles of sufficient mechanical integrity to withstand the aeration startup forces even with well settled CTA when the overall design of the primary and secondary oxidizer BCRs and of the digesters indicates that this baffle placement is useful for reducing over-oxidation to carbon oxides while attained target purities for PTA.

For mitigating or avoiding the above difficulties with settled solids during an outage, it is also preferred to provide a slurry drainage conduit leading from the bottom or very near bottom of the secondary oxidizer BCR to a reduced pressure outlet. More preferably, this lower drainage conduit connects to the suction of the pumps used for feeding slurry to the digester system. However, it is preferred for this alternate conduit to be fully obstructed by a closed valve during normal operation of the secondary oxidizer BCR.

A similar situation with outage sedimentation exists in the conduit conveying deaerated slurry from the primary oxidizer to near the base of the secondary oxidizer BCR. The operational situation in the smaller diameter slurry conduit is relatively more important than in the larger diameter BCRs. Whenever aeration is interrupted in the primary oxidizer BCR, the relatively tall slender conduit leading from the deaeration vessel may cease to be supplied with gas-lift slurry up to the elevation of the slurry side draw and deaeration vessel inlet. Soon thereafter, slurry flow in the conduit will cease when pressure balances with the secondary oxidizer BCR slurry inlet position. When this occurs for at least 2, 8, 16, 32 hours, it is preferred to cool the slurry remaining in the supply conduit by feeding inert gas near the lowest elevation of the conduit. It is preferred to feed enough gas to provide evaporative cooling of the remaining slurry by at least 10, 20, 30, 40°C below normal operating temperature, for this substantially retards cementitious binding of the CTA solids. It is more preferred to drain the supply conduit through the secondary oxidizer by its bottom outlet conduit, during or after drainage of the secondary oxidizer BCR. Optionally, a drainage conduit is provided from near the lowest elevation of the slurry supply conduit directly to the suction of the pumps used for feeding slurry to the digester system, but without passing through the secondary oxidizer BCR. Again, it is preferred for this drainage conduit to be fully obstructed by a closed valve during normal operation of the secondary oxidizer BCR.

Notwithstanding such provisions for re-starting operation after process outages and interruptions, it is preferred that many embodiments of the present invention be operated in a manner of substantially smooth and continuous flows.

Preferably, the time-averaged concentration of para-toluic acid in the liquid phase of the slurry withdrawn from the secondary oxidizer BCR is less than 50, 10, or 5 percent of the time-averaged concentration of para-toluic acid in the liquid phase of the slurry introduced into the secondary oxidizer BCR. Preferably, the time-averaged concentration of para-toluic acid in the liquid phase of the slurry introduced into the secondary oxidizer BCR is in the range of from 50 to 10,000, 100 to 6,000, or 500 to 5,000 ppmw. Preferably, the time-averaged concentration of para-toluic acid in the liquid phase of the slurry withdrawn from the secondary oxidizer BCR is less than 1,000, 200, or 60 ppmw. Preferably, the time-averaged concentration of 4-CBA in the liquid phase of the slurry withdrawn from the secondary oxidizer BCR is less than 50, 10, or 5 percent of the time-averaged concentration of 4-CBA in the liquid phase of the slurry introduced into the secondary oxidizer BCR. Preferably, the time-averaged concentration of 4-CBA in the liquid phase of the slurry introduced into the secondary oxidizer BCR is in the range of from 100 to 6,000, 200 to 4,000, or 400 to 3,500 ppmw. Preferably, the time-averaged concentration of 4-CBA in the liquid phase of the slurry withdrawn from the secondary oxidizer BCR is less than 500, 100, or 30 ppmw. Preferably, the time-averaged concentration of 4-CBA in the solid phase of the slurry withdrawn from the secondary oxidizer BCR is in the range of from 5 to 95, 10 to 90, 20 to 80, or 30 to 70 percent of the time-averaged concentration of 4-CBA in the solid phase of the slurry introduced into the secondary oxidizer BCR. Preferably, the time-averaged concentration of 4-CBA in the solid phase of the slurry introduced into the secondary oxidizer BCR is in the range of from about 100 to 15,000, 400 to 8,000, or 1,000 to 6,000 ppmw. Preferably, the time-averaged concentration of 4-CBA in the solid phase of the slurry withdrawn from the secondary oxidizer BCR is in the range of from 100 to 12,000, 300 to 8,000, or 800 to 4,000 ppmw.

The combination of primary oxidation and the secondary oxidation, if used, is referred to herein as initial oxidation and the CTA slurry product is referred to as initial slurry.

The catalyst system present in the liquid-phase of the digester feed stream is preferably a homogeneous, liquid-phase catalyst system capable of promoting oxidation (including partial oxidation) of para-xylene. The catalyst system may comprise one or more of cobalt, bromine, and manganese.

When cobalt is present in the catalyst system, it is preferred for the amount of cobalt present in the liquid-phase feed stream to be such that the concentration of cobalt in the liquid phase of the reaction medium is maintained in the range of from 300 to 6,000 parts per million by weight (ppmw), more preferably in the range of from 700 to 4,200 ppmw, and most preferably in the range of from 1,200 to 3,000 ppmw. When bromine is present in the catalyst system, it is preferred for the amount of bromine present in the liquid-phase feed stream to be such that the concentration of bromine in the liquid phase of reaction medium is maintained in the range of from 300 to 5,000 ppmw, more preferably in the range of from 600 to 4,000 ppmw, and most preferably in the range of from 900 to 3,000 ppmw. When manganese is present in the catalyst system, it is preferred for the amount of manganese present in the liquid-phase feed stream to be such that the concentration of manganese in the liquid phase of reaction medium is maintained in the range of from 20 to 1,000 ppmw, more preferably in the range of from 40 to 500 ppmw, most preferably in the range of from 50 to 200 ppmw.

The concentrations of the cobalt, bromine, and/or manganese in the liquid phase of the reaction medium may be expressed on a time-averaged and volume-averaged basis. As used herein, the term "time-averaged" denotes an average of at least 10 measurements taken equally over a continuous period of at least 100 seconds. As used herein, the term "volume-averaged" denotes an average of at least 10 measurements taken at uniform 3-dimensional spacing throughout a certain volume. The weight ratio of cobalt to bromine (Co:Br) in the catalyst system introduced into the oxidation reaction is preferably in the range of from 0.25:1 to 4:1, more preferably in the range of from 0.5:1 to 3:1, and most preferably in the range of from 0.75:1 to 2:1. The weight ratio of cobalt to manganese (Co:Mn) in the catalyst system introduced into the oxidation reaction is preferably in the range of from 0.3:1 to 40:1, more preferably in the range of from 5:1 to 30: 1, and most preferably in the range of from 10:1 to 25:1.

Optionally, the initial slurry of CTA from initial oxidation can be processed in a solvent exchange operation or a slurry thickness operation prior to being fed to digestion. When these optional operations are used, process objectives comprise adjusting the catalytic composition, e.g. liquid phase compositions of cobalt, manganese, bromine and water, removing additional amounts of liquid phase aromatic oxidation intermediates prior to digestion, removing liquid phase impurities not easily oxidized, e.g. 2,6-DCF and 2,7-DCF, to enhance the purity and whiteness of the digested PTA, and adjusting the mass fraction of solids in the slurry to alter the total volume of slurry and/or adjust the hydrodynamic properties of the slurry. After such treatment, the initial slurry is referred to herein as solvent-modified slurry of CTA.

When employing this optional solvent exchange operation, it is preferred to remove at least 40, 60, 80, 90 % of the solvent and soluble compounds found in the liquid phase of initial slurry. Thereafter, it is preferred to use cleaner solvent to provide a reconstituted slurry having mass fraction of solids as disclosed elsewhere herein. As used herein, the term "cleaner solvent" denotes solvent having a liquid phase concentration of total catalyst compounds that is less than the concentration of total catalyst compounds in the liquid phase of the slurry to which the cleaner solvent is added. Preferably, the cleaner solvent contains less than 90, 50, 10, or 2 weight percent of the liquid-phase concentration of total catalyst compounds and/or less than 90, 50, 10, or 2 weight percent of the liquid-phase concentration of total aromatic compounds compared to the liquid phase of the slurry to which the cleaner solvent is added.

When solvent exchange between primary oxidation and oxidative digestion is substantially eliminated in accordance with one embodiment of the present invention, it may be preferred for at least 30, 60, 80, or 95 weight percent of the initial liquid originally present in the initial slurry withdrawn from primary oxidation to be retained in the slurry subjected to oxidative digestion. Thus, it may be preferred for less than 70, 40, 20, or 5 weight percent of the initial liquid originally present in the initial slurry withdrawn from primary oxidation to be removed from the slurry subjected to oxidative digestion. Preferably, the weight ratio of cobalt, other catalyst compounds, and/or benzoic acid in the slurry entering oxidative digestion to the same compound in the initial slurry produced from primary oxidation is at least 0.3, 0.6, 0.8, or 0.95. More preferably, the weight ratio of cobalt, other catalyst compounds, and/or benzoic acid in the slurry exiting oxidative digestion to the same compound in the initial slurry produced from primary oxidation is at least 0.3, 0.6, 0.8, or 0.95. When oxidative digestion is carried out in multiple stages, the description in this paragraph can apply to any or all stages of oxidative digestion, most preferably including the last stage of oxidative digestion.

Accordingly, the slurry fed to the digester may be either initial slurry of CTA or solvent modified slurry of CTA and may hereinafter be referred to as digester feed slurry.

It is preferred that the solid CTA mass fraction in digester feed slurry is more than 0.10, 0.20, 0.30 and less than 0.50, 0.45, 0.40.

The amount of molecular oxygen required for oxidation of aromatic intermediates remaining in digester feed slurry provided according to the present invention is relatively small. It is preferred that the moles of molecular oxygen provided to all stages of digestion is less than the amount comprised in feeding 3, 2, 1 kg of compressed ambient air per 100 kg of digester feed slurry. In comparison, the feed rate of compressed ambient air to a primary oxidizer is often about 100 kg per 100 kg of initial slurry. In addition to relatively small molar and mass flow rates of air into digestion, the increased operating temperature of the digester system indicates a significantly greater operating pressure compared to initial oxidation, and this causes a further reduction in the volumetric feeding ratio for molecular oxygen supplied to the digester system. In combination, the small volumetric feeding rate of molecular oxygen to the digester system presents severe difficulties in providing enough gas mixing power in a digester BCR to satisfy process requirements comprising solids suspension, mass transfer of molecular oxygen from gas-to-liquid, and relatively rapid blending of the digester feed slurry where it enters the first digestion zone.

The mixing power provided by the rising gas within the reaction medium of the digester is closely approximated by the volume of flowing gas times the elevation head of the slurry through which the gas rises, since the slurry elevation head is typically much less than the overhead absolute pressure. As used herein, the term "gas mixing power" is defined as VdP and as being equal to the volumetric flow rate of gas rising within a digester vessel times the slurry elevation head through which it rises, with units appropriately converted to power units. As may be appreciated, many possible combinations of digester height and diameter are possible for a given total residence time of slurry, and many different operating pressures are possible. Accordingly, there are many possible results for the gas mixing power, but in all reasonable cases, the resulting gas mixing power is quite small within the digestion zones of the present invention. It is preferred that the gas mixing power summed in all BCR digester zones of the present invention divided by the slurry mass within these zones is less than 0.2, 0.1, 0.05 Watt/kg of slurry. Additional disclosure for the distribution of this limited amount of gas mixing power within various zones of the BCR digester system is disclosed elsewhere herein in discussion of superficial gas velocities and dimensions, i.e., height and width, of the various zones.

For reasons of carbon burn economy and overhead headspace flammability, it is generally undesirable to increase gas mixing power by feeding a greater excess of compressed ambient air than is needed as a bare minimum to effect removal of oxidation intermediates comprising para-toluic acid and 4-CBA. For reasons of capital equipment cost, compression energy cost and thermal energy cost, it is often undesirable either to increase or to reduce the mole fraction of molecular oxygen by adding or removing gaseous inert diluents comprising molecular nitrogen, carbon dioxide, carbon monoxide, molecular hydrogen, methane, methyl bromide and argon, inter alia. However, it remains within the ambit of the disclosures of this invention to feed to at least one digestion zone a compressed air stream that is either enriched or depleted in mole fraction molecular oxygen compared to the composition of ambient air.

The embodiments of the present invention obviate the need to provide mechanical shaft power agitation to the digester reaction medium when using the preferred range of CTA particle sizes and initial slurry composition, inter alia. However, the inventors have also discovered that adaptation of the bubbly flow gas power mixing to less ideal situations may be provided by surprisingly small amounts of supplementary mechanical shaft power agitation. For example, retrofitting the inventions herein to an existing digester vessel with a relatively flat 2:1 elliptical bottom head may require mild mechanical agitation within the lowest digestion zone to ensure that slurry solids do not sediment, stagnate and cementitiously agglomerate into large chunks of solids within the bottom head. When using supplementary mechanical agitation, it is preferred that the mechanical agitation power is less than about 0.2, 0.1, 0.05 Watt/kg of slurry averaged for the entire volume of digester reaction medium, and it is preferred that the combined total of gas mixing power and mechanical mixing power be less than about 0.30, 0,15, 0.10 Watt/kg of slurry averaged for the entire volume of digester reaction medium.

Digester slurry heating methods known in the art comprise oxidation of organic substrates other than para-xylene, e.g., oxidation of fed hexane to form carbon oxides and acetic acid; other chemical reactions, e.g. hydrolysis of acetic anhydride to form acetic acid; supplying a condensable solvent vapor to the slurry, e.g. water vapor and acetic acid vapor; and non-contact heating of the slurry and/or of the gaseous supply of molecular oxygen using a heat exchanger apparatus. Non-contact heating of the slurry is particularly preferred. Non-contact heating using an exchanger apparatus avoids the additional chemical complexity and/or vessel volume often needed when chemical heating or solvent vapor heating is used.

The exchanger heating unit for the digester slurry may be of any design suitable for the CTA slurry such that plugging and fouling of the unit are avoided. It must also be constructed of materials stable to the corrosive acetic acid mixtures. The heat exchanger surfaces may be located closely upstream of the first digester zone, which is preferably the top section of a BCR digester, being connected via slurry conduits between the initial oxidation and the digestion zones. It is preferred to provide at least 30, 60, 90, 100 percent of the total thermal duty for the digestion zone using at least one non-contacting heat exchanger apparatus on the slurry from initial oxidation before it is fed to the digestion vessel.

In one embodiment the CTA slurry heating unit may be of vertical tube-shell design such that the slurry passes through the tube side in a vertical upflow manner before entering the digester vessel. Optionally, heat exchanger surfaces can be located internally, i.e. within the digestion reaction vessel itself.

In another preferred embodiment, at least 25, 50, 75, 100 percent of the molecular oxygen supply for the first digestion zone, preferably the top or uppermost section of a BCR digester, is fed into the digester comingled with the digester feed slurry. Preferably, this molecular oxygen is combined with the digester feed slurry within 8, 2, 0.5 minutes after the slurry is first heated at least about 10°C above the temperature at the exit from initial oxidation. More preferably, at least 25, 50, 75, 100 percent of the molecular oxygen supply for the first digestion zone, preferably the top section of a BCR digester, is mixed with the slurry before the exit of an external heat exchanger, more preferably near the slurry inlet of an external heat exchanger. This close coordination in time between heating of the initial slurry and provision of molecular oxygen is important to prevent too much unreacted accumulation of liquid phase para-toluic acid and 4-CBA during the initial burst of CTA dissolution activity. For example, heating a CTA slurry from about 160°C to about 210°C increases the equilibrium mass fraction of dissolved TPA from about one-half percent of liquid phase mass to more than about two percent of liquid phase mass; and the smallest CTA crystals dissolve in just a few seconds after such slurry heating.

Importantly, the CTA particles obtained from the primary oxidation operation according to the present invention are characterized by particle morphologies that are smaller and more porous than CTA particles produced by many other para-xylene oxidation processes operating with different reactor mixing characteristics, residence times, volumetric oxidation rate intensities, pressure and temperature profiles, and solvent compositions, inter alia. In preferred embodiments, the solid CTA product obtained in the oxidation have a mean particle size in a range from 20 to 150 microns, preferably 25 to 100 microns and most preferably, 30 to 80 microns. The morphology of the particles is such that each particle is typically formed of a large number of small agglomerated particles and thus the CTA particles have a high BET (Braunauer-Emmett-Teller) surface area ranging from 0.6 to 4.0 $m^2$/g, preferably 0.8 to 3.0 $m^2$/g and most preferably, 0.9 to 2,0 $m^2$/g. This combination of particle size, surface area and agglomerated morphology leads to particles of high porosity, low density and low sedimentation velocity which are properties that facilitate the digestion process according to the present invention.

In comparison, CTA particles obtained by some oxidation methods are characterized by a mean particle size of approximately 180 to 220 microns and a BET surface area of 0.4 to 0.8 $m^2/g$. Such conventional particles have much less porosity and significantly higher apparent density measured in solvent liquid.

The physical properties of the CTA particles according to the present invention described above allow for effective and efficient digestion via the bubble column digestion method of the present invention because the small particles having greater surface area are more readily dissolved to release entrapped impurities to solution where oxidation may convert the impurity to product. Of equal importance, the smaller particle size and high porosity of the CTA particles allows for greater resistance to sedimentation in a bubbly-flow environment and thus the particle flow through a bubble column system may be controlled to sufficient residence time to allow purification as described.

The mass-averaged residence time of the solid phase and of the liquid phase, which may be different from each other owing to partial sedimentation in a digester BCR, are each in the range of from 10 to 480 minutes, preferably 20 to 360 minutes, and more preferably from 40 to 120 minutes, summed for all digester zones in series flow.

The residence time distribution of the solid phase is desirably improved using the present invention. Although there is a need for considerable liquid-phase mixing where the slurry first enters the digester, it is thereafter preferable for the solid phase to move through the digester system with a RTD more closely approaching plug flow RTD. The liquid phase mixing needed near the slurry entry relates to the initial release into the liquid phase of 4-CBA and para-toluic acid where the smaller particles in CTA slurry feed first enter the digester. Near the slurry feed location, it is desirable to provide sufficient convective mixing to control the local liquid phase demand for dissolved $O_2$, supporting the desirable aromatic oxidation in the liquid phase, and to control the local liquid phase concentration of 4-CBA and para-toluic acid that might be reprecipitated and buried as the solid particles enlarge. After meeting the reaction chemistry mixing and gas-to-liquid mass transfer requirements near the slurry feed location, it is thereafter preferable to control and lengthen the residence time in the digester for smaller particles with higher concentrations of solid 4-CBA and solid para-toluic acid. Such an RTD is promoted by various embodiments of the present invention comprising ranges of superficial gas rate, ranges of axial slurry velocity near the axial centerline of the vessel, ranges of particle size distribution, H to D ratios, and non-fouling baffling systems.

In the following disclosure, a notation is adopted wherein "t" is time; the residence distribution function of time is the Cumulative Mass Fraction (CMF) of a phase initially supplied to the reaction zone at time t=0 that then exits the reaction zone before time "t/$t_{avg}$"; where "$t_{avg}$" is the mass-averaged residence time determined according to the calculation described below and "t/ $t_{avg}$ " is "reduced time" meaning time divided by mass-averaged residence time. Reduced time is dimensionless. "CMF(t/ $t_{avg}$)" is the residence distribution function of reduced time. For example, CMF(0.2) is the cumulative mass fraction initially supplied to a phase of the reaction zone at time t=0 that then exits the reaction zone before a reduced time of 0.2. The mass average residence time ($t_{avg}$) of an aliquot of mass initially fed to an enclosure at time t=0 is calculated as [(t)*(mass of the aliquot exiting at time t)]/(total mass of the aliquot) integrated from time zero until at least about 99.9 percent of the mass of the aliquot has exited the enclosure. The units of $t_{avg}$ are simply any unit of time.

The oxidative digestion stage and/or series of oxidative digestion stages may be carried out in a single fluid enclosure or multiple enclosures with fluidic connection. In an embodiment of the present invention, it is preferred that at least one oxidative digestion stage, more preferably the stage wherein the at least 25, 50, 75, 100% of slurry from initial oxidation first enters, most preferably the top or uppermost section of a BCR digester, is sufficiently well mixed such that CMF(0.5) for only that stage/section is at least 0.20, 0.25, or 0.30 and such that CMF(1.5) is also less than 0.95, 0.90, 0.85 for each of the solid, liquid, and combined slurry phases. For this value of CMF, the normalizing time is the average residence time of slurry in the single digestion stage.

Further, it is preferred that the solid phase in digestion after the first oxidative digestion stage and/or in all BCR digester zones below the top zone approaches a plug flow (piston flow) RTD such that the CMF(0.5) is less than 0.35, 0.25, 0.20 for each of the solid, liquid, and combined slurry phases. For this value of CMF, the normalizing time is the total average residence time of slurry in all digestion stages not including the first, relatively well mixed digestion stage.

For the total of all digestion stages, whether in a single BCR or in multiple BCRs in series, it is preferred that the overall RTD has a CMF(0.5) of less than 0.35, 0.25, 0.18 and a CMF(1.5) of more than 0.80, 0.85, 0.90 and less than 0.98, 0.95 for each of the solid, liquid, and combined slurry phases. For this value of CMF, the normalizing time is the total average residence time of slurry in all digestion stages.

For achieving the desired mixing in the digestion stage where slurry is fed from initial oxidation, it is preferred that the time-averaged upwards velocity of slurry near the axial centerline near the mid-height of the BCR stage be at least 6, 8, 10 cm/s. The inventors have discovered that this can be achieved even in bubbly flow regime with superficial gas and superficial slurry velocity ranges disclosed herein providing that the BCR stage be of sufficient inside diameter. For example, uniform aeration with a superficial gas velocity of about 0.5 cm/s in small pilot scale BCRs with inside diameters up 30 cm achieves less than about 6 cm/s axial centerline slurry velocity upwards whereas the same superficial gas velocity in a commercially useful BCR with inside diameter of 2 meters or more achieves more than about 10 cm/s axial centerline slurry velocity upwards. Near the BCR wall there is perforce a compensating downwards flow of slurry.

BCRs with even larger inside diameters will circulate internally with even greater axial velocities without increasing the superficial gas velocity. This is a surprising result of the complex energy balancing of multi-phase natural convection comprising total energy provided by rising bubbles; slip, drag and local turbulence in the slurry near individual bubbles, induced overall convective flow of slurry, and the BCR wall drag forces.

For achieving the desired mixing in the later, preferably lower, stages of digestion where a more plug flow profile is preferred, it is preferred that the time-averaged upwards velocity slurry velocity at the axial centerline near the mid-height of each BCR stage be less than 20, 15, 10 cm/s. Near the BCR wall there is perforce a compensating downwards flow of slurry. This reduced circulation rate of slurry in the later, preferably lower, stages of digestion is provided by apt combinations of the disclosures herein comprising superficial gas rates, vessel inside diameters, digester stage heights, and gas feeding methods.

To obtain the desired balancing of hydrodynamic mixing coupled with suitable dissolution of molecular oxygen near the top of the digester zone where digester feed slurry first enters, which is preferably an uppermost zone of a BCR, the inventors have discovered that it is desirable for the superficial gas velocity there to be more than 0.1, 0.2, 0.4 cm/s and less than 8, 4, 1 cm/s. It is more preferred to feed undiluted compressed air while operating at a pressure and temperature combination in the digester that causes vaporized acetic acid solvent to comprise a significant portion of this disclosed superficial velocity.

To obtain the desired balancing of hydrodynamic mixing coupled with suitable dissolution of molecular oxygen in at least one digester zone after, preferably below, the digester feed slurry zone, the inventors have discovered that it is desirable for the superficial gas velocity in this later, preferably lower zone to be more than 0.01, 0.02, 0.04 cm/s and less than 4, 1, 0.2 cm/s. It is more preferred to feed undiluted compressed air while operating at a pressure and temperature combination in the digester that causes vaporized acetic acid solvent to comprise a significant portion of this disclosed superficial velocity. These are exceptionally small superficial velocities, and due care is required with the initial bubbling distribution to ensure adequate distribution of dissolved molecular oxygen along with the desired suspension of solids along with the desired RTD of the solids.

It is preferred that the superficial solid, liquid and slurry vertical velocities averaged over the entire volume of the digestion reaction medium are each more than 0.05, 0.1, 0.2 cm/s and less than 12, 8, 4 cm/s downwards. However, apt selections of the superficial gas rates and of slurry residence times in various digestion zones are typically more important.

The method of oxidative digestion according to the present invention substantially reduces the amount of at least one aromatic reaction intermediate compound. Preferably, the time-averaged concentration of para-toluic acid in the liquid phase of the slurry withdrawn from the later oxidative digestion stage is less than 50, 10, or 2 ppmw. Preferably, the time-averaged concentration of 4-CBA in the liquid phase of the slurry withdrawn from the later oxidative digestion stage is less than 50, 10, or 2 ppmw. Preferably, the time-averaged concentration of para-toluic acid in the solid PTA product withdrawn from the later oxidative digestion stage is in the range of from 1 to 1,000, 1 to 500, 5 to 125, or 10 to 60 ppmw. Preferably, the time-averaged concentration of 4-CBA in the solid PTA product withdrawn from the later oxidative digestion stage is in the range of from 1 to 1,000, 1 to 500, 10 to 250, or 20 to 125 ppmw. Preferably, the time-averaged concentration of 4,4'-DCS in the solid TPA product is less than 6, 4, or 2 ppmw.

The first embodiment of the present invention is a digestion method for purification of crude terephthalic acid as defined in claim 1. digestion

In a second embodiment the present invention includes an oxidative digestion system as defined in claim 3.

The oxidative digestion system is free of mechanical agitation.

The uppermost first digestion zone and the additional digestion zones may be structured as one bubble column reactor wherein the respective zones are vertically arranged with the lower additional zones sequentially beneath the uppermost zone.

In this embodiment the heated CTA slurry is injected into the uppermost zone of a column digestion reactor having multiple zones vertically arranged in the column reactor, with each zone being segregated by horizontally positioned baffle arrangements. It is preferred for the uppermost zone to comprise from 10 to 50 percent, 20 to 40 percent, 25 to 35 percent of the volume of all reaction medium in the vessel when at a normal operating level. It is preferred that the top height of the vessel provide an additional level control surge volume and gas disengaging volume that is equal to at least 10, 15, 20 percent of the volume of all reaction medium in the vessel when at a normal operating level, and it is also preferred that the clearance from the normal operating level to the off-gas outlet nozzle is at least 1 meter, more preferably 2 meters above the maximum operating level. Although the superficial gas velocity is very low and gas disengagement is typically not problematic, any type of spray reflux and mechanical impingement disengagement devices known in the art may be used in the ullage to further suppress foaming and misting entrainment of slurry in digester off-gas.

Owing to the quite low gas mixing power and total mixing power employed in lower additional digestion zones of the present invention, it is preferred to use a conically shaped bottom vessel head, preferably with an included angle at the bottom inverted apex of the cone of at least 40, 60, 80 degrees and less than 140, 120, 100 degrees, preferably with a slurry outlet nozzle discharging vertically downwards from the bottom apex of the inverted cone.

The baffle structure separates each zone from a zone directly vertically beneath and vertically above and each baffle unit is structured to allow upward passage of gas bubbles and downward passage of particles. The uppermost zone has only a lower baffle while the lowermost zone has only an upper baffle. Each intermediate zone has both a lower baffle and an upper baffle. The baffle unit may comprise a tray having multiple inverted shaped sloped surfaces with multiple open areas.

Preferably, the open area of the baffle or baffles is in the range of from 5 to 75 percent, more preferably in the range of from 10 to 35 percent of the total horizontal area of the baffle.

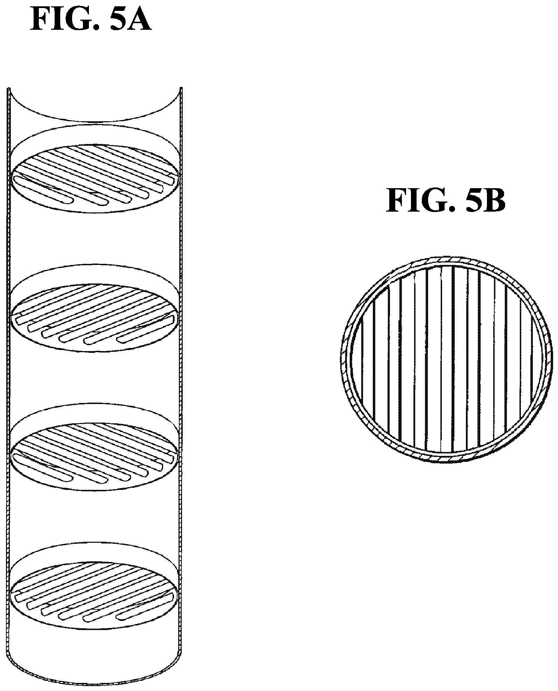

The baffles are resistant to fouling. Baffles having a significant amount of near-horizontal upwardly-facing planar surface area may be prone to fouling where solids build up on the upwardly-facing surfaces of the baffles, and as the amount of solids deposited on the baffles increases, chunks of the precipitated solids may dislodge from the baffles and fall towards the bottom of the reactor. These chunks of dislodged solids can block apertures in the baffles causing a deterioration of the solids flow pattern and residence time distribution within the reactor. These chunks of solids can also build up in the bottom of the reactor and can cause a number of problems including, for example, inhibition of slurry discharge out of the bottom of the digester. In one embodiment the baffle presents no upwardly-facing planar outer surfaces and may be constructed from piping materials having a circular cross section. A schematic drawing of an example of such a baffle is shown from different perspectives in Figs. 5A and 5B. In Fig. 5A the baffle is viewed from a horizontal perspective while in Fig. 5B the baffle is viewed from a vertical perspective.

Unless otherwise defined herein, an upwardly-facing surface is a surface having a normal vector projecting above horizontal. In another embodiment, a small amount of substantially planar surfaces may be utilized so long as less than 50 percent of the total upwardly-facing exposed outer surface area of the baffle comprises substantially planar surfaces inclined less than 35° from horizontal. It is further preferred for the upwardly-facing exposed outer surfaces of the baffle to have a substantially smooth finish so as to further resist fouling. Preferably, at least a substantial portion of the upwardly-facing exposed outer surfaces of the baffle or baffles have a surface roughness less than 125 micron RMS, more preferably less than 64 micron RMS, and most preferably less than 32 micron RMS. Electropolished finishes and smooth "2B" mill rolled finishes are particularly useful.

In another embodiment, the baffle may comprise a plurality of elongated individual baffle members. In this embodiment, each baffle member is formed of an L-section member and presents a generally inverted V-shaped upwardly-facing exposed outer surface. The number, spacing, and orientation of angle iron baffle members can be substantially the same as described above for cylindrical baffle members described above.

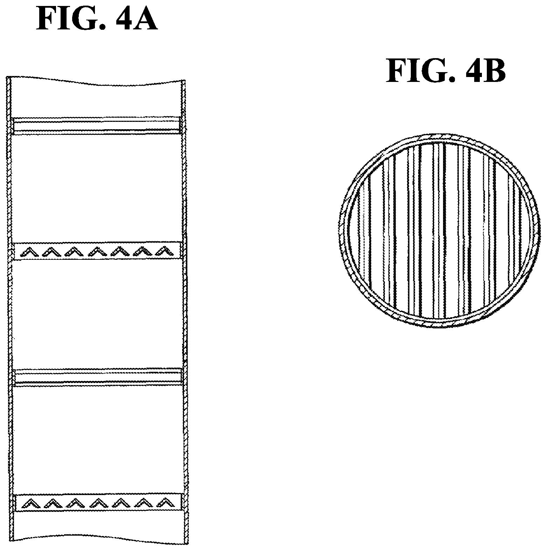

In one preferred embodiment of the invention the baffle is structured as a series of horizontal inverted "V" sections separated by open gaps. The slope of each leg of the inverted "V" is about 45° thus allowing downward moving particles to pass through the baffle in a gentle flow without plugging the baffle. At the same time upward moving air bubbles can simultaneously pass through the baffle. A schematic drawing of such a baffle is shown from a horizontal perspective in Fig. 4A and from a vertical perspective in Fig. 4B. In the embodiment shown in Fig. 4A the longitudinal axis of each parallel aligned segment is varied from one baffle to another. In Fig. 4A the alternating baffle segments are oriented at 90° angle relative to one another. Alternative angle arrangements may be employed to vary solids and gas flow parameters as described elsewhere in this description.

Figs.5A and 5B show a baffle comprised of a group of parallel arranged spaced pipes which in series in the BCR column are oriented at angles relative to one another from one baffle to the next. Although Fig. 5A shows the relative orientation to be perpendicular, other relative angles may be arranged in order to vary solids and gas flows.

In another preferred embodiment, the baffle may be structured as a series of adjacent cones having a conical angle of approximately 45° and each cone ending in a hole.

In the baffle structure design the open area is 5 to 75 percent of the total area of the baffle horizontal area, more preferably in the range of from 10 to 35 percent. Such design is necessary to prevent back-mixing between the vertically adjacent zones while still allowing the downward flow of particles and upward flow of air bubbles.

In addition to the air inlet of the first digestion zone at least one of the lower additional digestion zones are equipped with an air inlet located in a lower portion of the zone. Each intermediate digestion zone may optionally comprise an air inlet. In one embodiment of the present invention all zones of the digester are equipped with air inlets.

It is preferred that each air supply inlet located in a digestion zone comprises a suitable sparger system such that the air is evenly dispersed into the zone to create a "bubbly flow" wherein the released gas bubbles are relatively widely spaced horizontally and travel upwards with a relatively small amount of bubble coalescence and breakup. With apt design of a gas sparger, it is often possible to sustain bubbly flow to a superficial gas velocity of about 4 cm/s. A BCR having a superficial gas velocity of about 4 cm/s upwards to about 10 cm/s is often operating in a transition regime of the bubble column hydrodynamics. The boundaries of this transition regime are somewhat variable with fluid properties and system geometry. From a superficial gas velocity of about 10 cm/s and upwards, bubble column hydrodynamics move increasingly into a "churn turbulent" regime wherein bubbles chaotically coalesce, break-up and form bubble swarms. In one embodiment of the present invention the sparger may be constructed in a star or wheel-spoke structure in order to effectively provide the bubbly flow. The construction of the sparger system with regard to hole size, spacing, angle, etc. is conventionally known and may be commercially engineered and obtained.

Each air sparger system may be equipped with a "flush system" such that steam and/or high pressure (HP) aqueous acetic acid liquor or vapor is mixed with the air and passed through the sparger to prevent solid fouling of the sparger. An additional benefit of continuous steam flushing is to avoid excessive oxidation rates when aqueous acetic acid comprising catalyst components weep or otherwise flow from the slurry to the inside of the sparger assembly. Of course, the added mass and energy of any flush fluid must be considered in the local and overall plant energy and mass balances.

The heated CTA slurry is passed into the uppermost digester zone through an inlet located above the air sparger of the first or uppermost zone. The CTA inlet and air sparger of the first or uppermost zone are co-designed so that maximum mixing and co-mingling of the CTA particles and air bubbles take place when the heated particles enter the uppermost zone. As described previously, at the higher temperature the terephthalic acid dissolves from the surface of the particles and the 4-CBA and p-toluic acid entrapped in the particle are released into solution where interaction with the oxygen of the air leads to further oxidation. This process is greatly enhanced with the small, porous high surface area particles of low density of the present invention and the release rate of partial oxidation impurities is particularly rapid directly subsequent to slurry heating.

The air flow rate provides or initiates a significant portion of the mixing energy in the 3-phase chemical system present in the bubble column digester. The total mixing energy in the first or uppermost zone is principally a function of the air flow rate and the dispersive force of the particles as the CTA slurry is injected into the zone. Height and diameter values of the zone are important engineering design parameters that must be determined in consideration of the mixing energy per unit volume necessary. When the diameter is made smaller, the height needed for the same volume of reaction medium obviously becomes greater. In addition, the VdP gas mixing work also becomes greater because the elevation head of slurry traversed by the gas flow volume increases. However, the end-to-end height of the zone also affects the mixing circulation time, as does the non-linear interaction of column diameter with superficial gas velocity in setting the axial rising velocity of slurry at the axial centerline. The mixing effects provided by the air supply and the particle insertion energy in this zone must achieve the desired suspension or movement of the particulate solid phase so that sufficient residence time in the zone is achieved.

The amount of air fed to the uppermost or first digestion zone, comprising air fed with digester feed slurry, air rising from lower digester zones and air fed directly to the first or uppermost digester zone, must supply an amount of oxygen that is stoichiometrically in excess of the amount required to oxidize the 4-CBA and p-toluic acid released in the first or uppermost zone to terephthalic acid, must support convection movement of the CTA slurry, and must avoid providing a large excess of molecular oxygen. The principle cost of excessive molecular oxygen in all zones of the digester is typically excessive "carbon burn" of acetic acid and other oxidizable compounds present in the CTA slurry in contrast to the mechanical compression energy to provide the air.

The inventors have recognized that under the disclosed digester conditions, providing gas rising under bubbly flow provides an oxygen dissolution rate into the liquid phase that may be made sufficiently rapid to support the desired oxidation of dissolved oxidation intermediates such as 4-CBA and para-toluic acid. This provision of dissolved oxygen supply is without need to provide greater than specified amounts of gas and total mixing power to promote the "kLa" mass transfer coefficient. In bubble columns, the value of kLa is closely associated with the bubble volume fraction, and bubble volume fraction is more strongly increased by superficial gas velocity in the bubbly flow region than in the transition and churn turbulent regions. The provision of sufficient dissolved molecular oxygen also depends on the demand rate for this oxygen, and the disclosures herein comprising the preparation and provision of digester feed CTA slurry, the preferred heating amount for digestion, and time lag between heating and combination with fresh supplies of air are also important factors in maintaining apt concentrations of dissolved molecular oxygen without resorting to greater gas and total mixing powers or greater amounts of total air feeding.

The total demand for molecular oxygen for the desired conversion of aromatic oxidation intermediates to TPA product in the digester is surprisingly small. Typically, the stoichiometric oxygen demand in digestion is much less than 1% of the demand for molecular oxygen in the primary oxidation. Of course, the molecular oxygen consumed by undesirable carbon burning reactions in digestion is additive, but the total the required flow of molecular oxygen in digestion is still relatively small and typically less than 0.5% of molecular oxygen supplied to initial oxidation. As a result, a digester BCR sized for apt residence time and apt flow of molecular oxygen is typically operating quite low within the homogenous, bubbly flow regime of BCR hydrodynamics.

For the purpose of obtaining apt kLa mass transfer rates in combination with apt convective hydrodynamics, it is preferred that air feeding rates, vessel geometry, system temperature and pressure, superficial gas velocities, and slurry composition be selected within ranges disclosed elsewhere herein such that the time-averaged, area-averaged gas hold-up fraction near the top of the first, uppermost zone of the digester BCR be more than 0.5, 1.0, 1.5 percent and less than 6, 4, 2 percent. In at least one subsequent secondary digester BCR zone, it is preferred that the time-averaged, area-averaged gas hold-up fraction be less than 2, 1, 0.5 percent.

In correspondence with oxygen dissolution, the solvent may pass into vapor phase within the air bubbles and the rising vapors lead to a vapor head space containing air and organic vapors. The partial pressure of oxygen may be quite high in the disclosed process pressure ranges. If not controlled, the head space vapors may become potentially explosive as the oxygen content approaches about 8 volume per cent, measured after condensing out solvent vapors and thereby converting the oxygen measurement to a "dry basis" measurement that corresponds with, but is different than, the actual oxygen concentration at process conditions in the head space and off-gas. Thus monitoring the oxygen content of the head space and controlling the oxygen content to less than 6 volume % dry basis is preferred for reasons of process safety.

In addition, it is preferred for reasons of process economy relating to carbon burn and air supply cost to adjust the dry basis molecular oxygen remaining in digester headspace off-gas to be preferably less than 4 volume % more preferably less than 2 volume % and most preferably less than 1 volume %, all dry basis values. This provides an efficient and effective method to control the desired conversion of para-toluic acid and 4-CBA to PTA in balance with minimizing carbon burn. When all digestion zones are arranged vertically in a single BCR, this balancing is performed beginning with an excess air supply to each zone and about 4 volume percent oxygen dry basis in the overhead off-gas. Then the air supply rate to the lowest zone is decreased until the produced concentration of 4-CBA in PTA begins to rise undesirably. Moving upwards zone by zone, the air supplies to various zones are similarly titrated for final 4-CBA concentration in produced PTA versus reduced air supply rate. In this way the excess carbon burn can be minimized while maintaining target PTA purity. However, there are typically small variations in all process variables such that the most strictly minimized air supply rate profile may not provide good operational stability, and so small increases in excess air may be provided to one or more zones to arrive at the cumulative effect on overhead off-gas oxygen composition according to the above preferred ranges.

As indicated above the arrangement of the slurry inlet and air sparger in the uppermost digester zone is designed to maximize interaction of the particles and oxygen immediately upon entry of the heated CTA slurry into the zone. Mixing and flow within each zone is controlled by the design of the particular zone.

Preferably, the digester feed slurry is released into the first or uppermost zone near the axial centerline of the vessel at a position somewhat above the baffle at the lower portion of the zone. As described elsewhere herein, bubbly flow in larger diameter bubble columns produces a significant natural convectional axial flow circulation. The liquid/slurry phase within a central core of the vessel flows upwards, and this is counterbalanced with a downwards flowing outer annulus region. In bubbly flow, this core annulus inversion point of the liquid/slurry flow is typically at about 0.7 times the vessel radius. Preferably, the inlet of the CTA slurry is designed so that the slurry is significantly dispersed horizontally within the central core, but without being significantly projected into the outer down flowing region. Preferably, the slurry inlet may be positioned at least 1 or 2 meters above the lower baffle with the air sparger located between the slurry inlet and lower baffle. This positioning is designed to reduce the fraction of digester feed slurry particles that are passed more quickly downward to the baffle, through the baffle and into the zone beneath.

A preferred total residence time in the first digestion zone may be from 10 minutes to 60 minutes, preferably 15 minutes to 50 minutes and most preferably from 20 to 40 minutes.

It is preferred that the height of the first, uppermost zone be less than 30, 20, 10 meters. This provides an end-to end mixing time of the first uppermost zone aptly matching recrystallization and other chemical kinetics with the axial core-annulus circulating velocity profile made available by the very small amounts of gas mixing power and total mixing power disclosed herein. In order to increase the gas mixing power provided by the small volumetric flow rates of gas (V) disclosed herein, it is preferred to that the first uppermost zones have a height that is more than 1, 2, 4 meters, thereby increasing the dP term in the VdP gas mixing power. In order to increase the superficial gas velocity and natural convection axial slurry circulation velocity profile made possible with the small volumetric flow rates of gas disclosed herein, it is preferred that the first uppermost zone have an inside diameter that is less than 16, 12, 10 meters. In order to increase the natural convection axial circulation velocity profile by avoiding too much wall drag resistance from the vessel wall, it is preferred that the first uppermost zone have an inside diameter that is more than 0.5, 1.0, 1.5 meters. To balance these competing hydrodynamic objectives, it is preferred that the first uppermost zone have a ratio of reaction medium diameter to reaction medium height of more than 0.5, 1.0, 1.5 to 1 and less than 16, 8, 4 to 1.

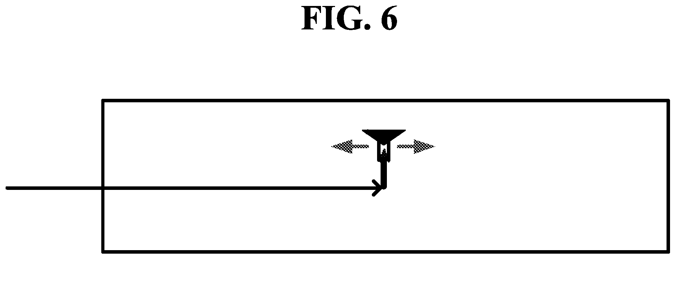

In one embodiment the inlet of the slurry supply conduit to the first or uppermost digestion zone may include a deflector unit attached to the top of a vertical inlet pipe. The deflector may be in the shape of an approximately horizontal flat impingement plate, an inverted impingement cone or any shape that improves the horizontal distribution of slurry outward from the termination of the supply conduit, more preferably without significant deflection downward toward the baffle in the lower portion of the first or uppermost digestion zone. A schematic diagram of one embodiment of a deflector unit is shown in Fig. 6 where an inverted cone deflector is positioned atop a vertical CTA entry pipe. The angle of deflection may be varied in order to optimize slurry distribution for a given entry flow rate and other parameters associated with the equipment design. As indicated above the energy imparted to the system by such deflection adds to the mixing energy in the uppermost zone and improves the random distribution of the particles within the core region of the zone.

Optionally, at least one flow eductor apparatus may be provided to use the kinetic energy of entering digester feed slurry to induce additional circulation of the reaction medium near the end of at least one digester feed slurry supply conduit.

It is preferred that at least 25, 50, 75, 100 percent of the mass flow rate of digester feed slurry exiting supply conduit openings into a digester feed zone is provided with a superficial velocity at respective conduit openings of more than at least 1, 3, 5 m/s and less than 70, 50, 30 m/s.

Optionally, the digester feed slurry may be split into two or more approximately equal portions of mass flow rate and fed at separated axially positions. Preferably, these slurry feed positions are each separated axially by at least 0.5, 1.0, 1.5 times the inside diameter of the bubble column digester vessel at the elevations where digester feed slurry is introduced. Certain combinations of digester feed slurry composition and mass flow rate may indicate selections of air feed rates and vessel diameters that are not adequately mixed with respect to controlling the local liquid-phase compositions of dissolved para-toluic acid, 4-CBA, and molecular $O_2$ near the slurry feeding openings. In such cases, a splitting of digester feed slurry flow and axial separation of digester feed slurry introduction openings reduces the mixing difficulties when employing natural convection bulk circulation of reaction medium provided principally by gas mixing power in the bubbly flow regime. The splitting of the digester feed slurry flow may comprise flow measurement and variable flow control elements situated in supply conduits, supply conduit design geometry such as symmetry of frictional pressure drop with adjustment for differences in elevation head, and other means known in the art.

The off-gas from the uppermost zone may be passed to a system to recover solvent mass and/or thermal and/or mechanical shaft energy, as is known in the art, or the off-gas may be combined with off-gas from the primary and/or secondary oxidation systems for recovery of solvent mass and/or thermal and/or mechanical shaft energy.

In the zones beneath the uppermost digester zone, the air feeding rate and air feeding inlet design structure are such that less core-annulus axial circulation is induced as the slurry bulk superficial flow of slurry continues downward. This is beneficial from a process efficiency perspective, because it is desirable for the particles to pass through each of these lower additional digestion zones in an approximately plug-flow manner. However, even small amounts gas superficial velocity deeply within bubbly flow still induce axial circulation that is significant relative to residence time in PTA digester BCRs of commercially relevant size. Accordingly, it is preferred to use non-fouling baffles to create axial subdivisions of the digester volume after, more preferably below, the digester zone receiving digester feed slurry. These baffles interrupt the continuity of axial circulating currents near and across the baffles, and in some cases of zone height and diameter, the baffles may also suppress the maximum velocity of the induced core-annulus circulating currents in between two baffles. Addition detailed disclosure on the mechanical design and physical placement of these baffles is provided elsewhere herein.

As previously indicated, the major portion of the air fed to the digester is fed to the first digestion zone. Thus from 50 to 90 mass % of the air injected into the digester may be injected into the first zone. The remainder may be split between the second and additional digestion zones in equal portions or in varying ratios adjusted as disclosed elsewhere herein.

As described above, an advantage of the use of bubble column reactors is the reduction of capital equipment costs and on-going energy requirement associated with the operation and maintenance of mechanical agitators. In comparison to prior technology for oxidative digestion, e.g. U.S. 7,393,973, the embodiments of the present invention may reduce installed capital costs for a CTA digester system, which comprises very large vessels, equipment and piping made with titanium, by upwards of $10,000,000 in a facility sized at present capacities of about 1,000 tons per year of TPA. This savings is a result of combining the prior preference for multiple vessels in series into a single vessel with smaller total volume, without mechanical agitation, and without separate heating means for the separate vessels. The savings in electrical motor power is in excess of 300 kilowatts. The savings in digester slurry heating duty is in excess of 10 percent by avoiding the use of vaporized solvent without compromising the ability to purify CTA at favorably low temperatures. The savings in design capacity and in operating cost for a recycle filtrate purge purification system is about 10 percent.

U.S. 7,393,973 discloses that the mechanical energy to drive CSTR digestion reactors is from 0.2 to 0.8 kilowatts per cubic meter ($kW/m^3$) of the digestion reaction slurry. The present inventors have calculated that in an existing conventional CSTR digester system with two equally sized vessels in series, the total of mechanical agitator energy consumption and gas mixing power is approximately 0.3 Watt/kg. In contrast the energy power of the BCR digester of the present invention processing the same digester feed is estimated to have only gas mixing power amounting to about 0.02 Watt/kg. Thus the energy consumption cost of the method according to the present invention is significantly less than that of CSTR type methods.

The total height of the zones arranged for plug flow-like particle sedimentation beneath the uppermost digestion zone may vary from 1.0 to 5.0 times the length of the uppermost zone, preferably from 1.5 to 4 times the length and most preferably, at least twice the length of the uppermost zone. The ratio of the total height of the lower additional digester zones to the diameter of a lower additional digester zone may be from 2/1 to 12/1, preferably 3/1, to 8/1 and most preferably, 4/1.

The total number of lower additional digestion zones is determined by the number of horizontal baffles placed in the column to segregate individual zones. The minimum number of baffles in the digester is one wherein the column would contain one first uppermost more-well-mixed digestion zone and one lower additional more plug flow digestion zone. Preferably, the column contains more than one baffle and less than 10 baffles, preferably from 2 to 8 baffles and most preferably from 3 to 6 baffles providing a corresponding number of lower additional digester zones having reduced superficial gas velocity relative to the first uppermost zone.

In one embodiment according to the invention the diameter of the digester column is constant from top to bottom. In other embodiments the column may be constructed such that the diameter varies from zone to zone. For example, the diameter of the lower zones may be less than the diameter of the uppermost zone such that a high height/diameter (H/D) ratio is achieved in order to promote controlled plug-flow passage through the secondary zones with minimum gas-lift requirement. On the other hand the uppermost zone may be structured to have effective convectional mixing with least gas-lift power requirement.

As described previously, the CTA particles of the slurry obtained from the primary oxidation system are non-uniform in shape and particle size distribution. Due to Ostwald ripening occurring in the digester, the particle size distribution may typically enlarge and narrow as the solids progress through the digester zones. The inventors have recognized that in order to effectively conduct the digestion of the present invention, the relationship between particle size and morphology and sedimentation rate must be considered. Digestion temperature may be an important parameter within such consideration. The inventors have learned that higher digestion temperatures lead to increased particle size. For example, digestion at temperatures of approximately 240 to 260 °C may result in particles of approximately 200 microns, and particles of such size have a much greater rate of sedimentation even when sedimentation is hindered by being in a slurry with fraction of solids as disclosed herein.

Accordingly, in preferred embodiments of the invention the temperature of the digestion in each of the zones is in a range of approximately 180 to 230 °C, preferably, 190 to 220 °C and most preferably, 200 to 210 °C.

Optionally, the first uppermost digestion zone may be operated either at higher temperature or at lower temperature than at least one of the subsequent digestion zones by a temperature differential of 40, 20, 10, 5 °C. This temperature differential may be useful in balancing the removal of oxidation intermediates, e.g. 4-CBA, with carbon burning reactions and/or resulting particle size distribution. The heating of a subsequent zone may be accomplished using any of the heating methods disclosed herein. Cooling of a subsequent zone may be effected by adding a mass of cooler solvent liquid, by heat exchange surfaces, preferably mechanically scraped surfaces, using a cooling fluid, and by evaporative cooling comprising gas feeding and pressure reduction, providing that other aspects of the invention pertaining to superficial gas rates, system pressures, and operating temperatures are maintained in disclosed ranges.

Preferably, the solid PTA product formed by digestion using one or more of the inventive embodiments disclosed herein essentially comprises particles having a mean particle size, which is D(4,3), of at least 30 microns, more preferably in the range of from 35 to 200 microns, still more preferably in the range of from 40 to 160 microns, and most preferably in the range of from 45 to 120 microns. Preferably, the solid TPA product essentially comprises particles having a measured value of D(v,0.1) in the range of from 5 to 60 microns, more preferably in the range of from 10 to 50 microns, and most preferably in the range of from 15 to 40 microns. Preferably, the solid TPA product essentially comprises particles having a measured value of median particle size, which is D(v,0.5), in the range of from 25 to 160 microns, more preferably in the range of from 30 to 100 microns, and most preferably in the range of from 35 to 80 microns. Preferably, the solid TPA product essentially comprises particles having a measured value of D(v,0.9) in the range from 40 to 300 microns, more preferably in the range from 60 to 250 microns, and most preferably in the range from 80 to 200 microns. Preferably, the solid TPA product essentially comprises particles having a measured value of particle size relative spread in the range from 0.6 to 5.0, more preferably in the range from 0.9 to 4.0, and most preferably in the range from 1.2 to 2.5. Preferably, the solid TPA product essentially comprises particles having an average BET surface area less than 0.25 square meters per gram (m2/g), more preferably in the range of from 0.005 to 0.2 m2/g, and most preferably in the range of from 0.01 to 0.18 m2/g.

Owing to the very low gas and total mixing power embodied in the present invention, it is preferred that the combination of solid particles size distribution, fraction of solids in the slurry, and liquid medium composition, temperature and pressure are selected to provide the following sedimentation rate ranges. It is preferred that the unhindered sedimentation rate of the mean D(4,3) particle size of the PTA particles leaving the digester zones be less than 120, 100, 80 meters per hour. In lieu of physical measurement at actual process conditions using separated individual particles of apt size, this unhindered sedimentation rate may be calculated using Stokes Law with an assumed spherical particle shape as is known in the art. In addition, it is preferred that the hindered sedimentation rate of the slurry of the PTA particles leaving the digester zones be less than 30, 20, 10 meters per hour. In lieu of physical measurement at actual process conditions, this hindered sedimentation rate may be calculated by multiplying the above calculated Stokes Law terminal velocity sedimentation rate of the mean D(4,3) particle size times a slurry concentration correction factor, which is often referred to as the Richardson and Zaki method, where epsilon is the liquid volume fraction in the slurry:

$$\text{Hindered sedimentation rate} = \text{Stokes Law rate} * epsilon\char`^2 * epsilon \char`^2.65$$

An exhaust gas outlet at the top of the uppermost zone may be equipped with an oxygen monitoring system to determine oxygen content. The exhaust outlet may transfer the exhaust gas to a condenser system to recover evaporated solvent and other volatile organic materials present or may recycle the exhaust gas to the primary oxidation system.

The bubble column digester according to the present invention may have from 1 to 5 lower secondary zones beneath the uppermost zone. The total height of the column may be from 16 to 40 meters, preferably 20 to 30 meters and most preferably 22 to 28 meters. The diameter of the column may be from 1.0 meter to 8.0 meters, preferably 2.0 to 6.0 meters and most preferably approximately 3.0 to 5.0 meters.

The vertical length of the uppermost zone may be from 4 to 12 meters, preferably 6 to 10 meters and most preferably approximately 8 meters. The vertical lengths of the lower digestion zones may be from 2 to 6 meters and actual design will be based on maximum plug flow character in the lower zones.

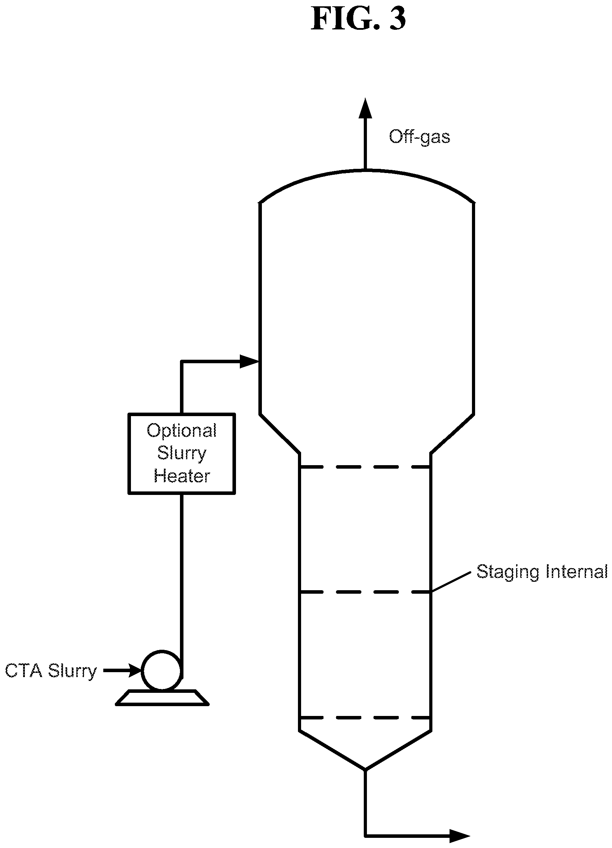

Each zone may have the same diameter or according to special design parameters, the diameter may be varied from zone to zone. For example as shown in Fig. 3, the diameter of the uppermost digestion zone may be greater than the diameter of the lower zones. The number of zones is determined by the number of baffles placed in the column. No mechanical stirring devices are necessary in any of the zones. The structure of the baffles was provided previously in description of the method embodiment.

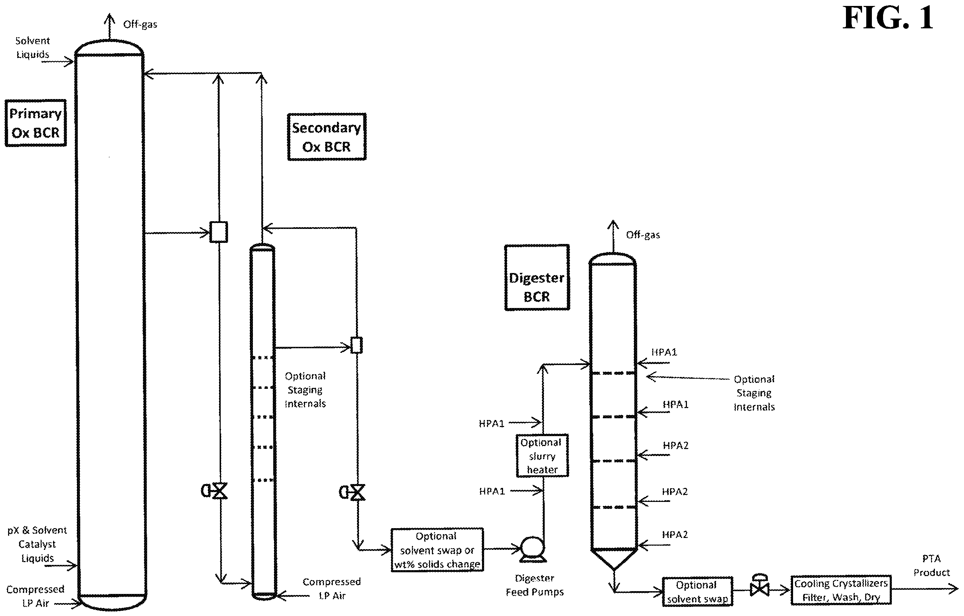

A schematic PTA production system according to the single BCR embodiment of the present invention is shown in Fig. 1. According to the production flow stream CTA slurry obtained from a primary oxidation system containing a primary BCR oxidation unit and a secondary BCR oxidation unit is injected into an uppermost zone of a BCR containing five vertically arranged zones segregated by horizontal baffles. Optional placement of air injection units for the first zone are indicated by HPA1 locations while HPA2 locations are optional air injection units for the second and subsequent digestion zones vertically beneath the first digestion zone. In each case one or more of the HPA1 and HPA2 inlets may be present in the locations indicated.

Alternatively, in another embodiment, two or more BCR reactors may be employed wherein the first vessel corresponds to the uppermost digester zone first receiving digester feed slurry and the second BCR reactor receives process slurry from the first BCR. As the capital cost for a BCR is significantly less than a CSTR, such a two column system is cost effective.

According to this embodiment of the present invention the sequential zones may be separated into two or more bubble columns in series, the first BCR being structured for more convectional mixing as described above for the first uppermost digestion zone of the single BCR system and the subsequent one or more BCRs being structured to have RTDs more closely approaching plug-flow-like passage of the particle slurry as described for the lower additional digestion zones. Thus the present invention also includes a bubble column digestion system as defined in claim 12. According to this embodiment the disclosure of digester feed and oxygen gas inlet structure and arrangement for the first uppermost zone of the single BCR embodiment is also applicable to the structure and arrangement of the first BCR of the multiple BCR embodiment. Likewise the disclosure of structure and arrangement for the second or more zones vertically beneath the first uppermost zone of the single BCR system is applicable to the second and optional subsequent BCRs of the multiple BCR system.

The first unit is structured for optimizing convectional flow having a height of at least 8 meters and a H/D ratio of 4 or less, preferably the H/D ratio is 3 or less and most preferably the H/D ratio is 2.

The first BCR unit is engineered to have convectional flow wherein a central core of upward bubbly flow is counterbalanced with an outer down-flowing annulus region. The inlet of the CTA slurry is designed so that the slurry is quickly dispersed within the central core without being projected into the outer down flowing region where oxygen content would be reduced. The slurry inlet may be positioned in the lower quadrant of the BCR approximately 1 or 2 meters above the air sparger located between the slurry inlet and the slurry outlet at the bottom of the BCR. As described above, this positioning is designed to maximize particle flow upwards in the central core region where interaction with oxygen is greatest before the particles move downward to the slurry outlet.

The sparger may be constructed in a star or wheel-spoke structure in order to effectively provide the bubbly flow. The construction of the sparger system with regard to hole size, spacing, angle, etc. is conventionally known and may be commercially engineered and obtained.

The air sparger system may be equipped with a "flush system" such that steam and/or high pressure (HP) acid wash is mixed with the air and passed through the sparger to prevent solid fouling of the sparger.

An exhaust gas outlet at the top of the BCR unit may be equipped with an oxygen monitoring system to determine oxygen content. The exhaust outlet may transfer the exhaust gas to a condenser system to recover evaporated solvent and other volatile organic materials present or may recycle the exhaust gas to the primary oxidation system.

A baffle such as described above may be located beneath the air sparger and slurry outlet.

The slurry outlet is connected to a highest zone of a second BCR unit via a transfer line, optionally having a transfer pump unit. As described, the second BCR unit is designed for plug flow and may have from 1 to 5 horizontally segregated zones distinguished by baffles as previously described. At least one zone of the second BCR unit is equipped with an air inlet and optionally each other zone may independently be equipped with an air sparger. The total height of the second BCR unit may be from 16 to 40 meters, preferably 20 to 30 meters and most preferably 22 to 28 meters. The diameter of the column may be from 1.0 meters to 8.0 meters, preferably 2.0 to 6.0 meters and most preferably approximately 3.0 to 5.0 meters.

The vertical lengths of the individual plug-flow zones may be from 2 to 6 meters and actual design will be based on optimum plug flow character in the zones.

Each zone may have the same diameter or according to special design parameters, the diameter may be varied from zone to zone. The number of zones is determined by the number of baffles placed in the column. No mechanical stirring devices are necessary in any of the zones.

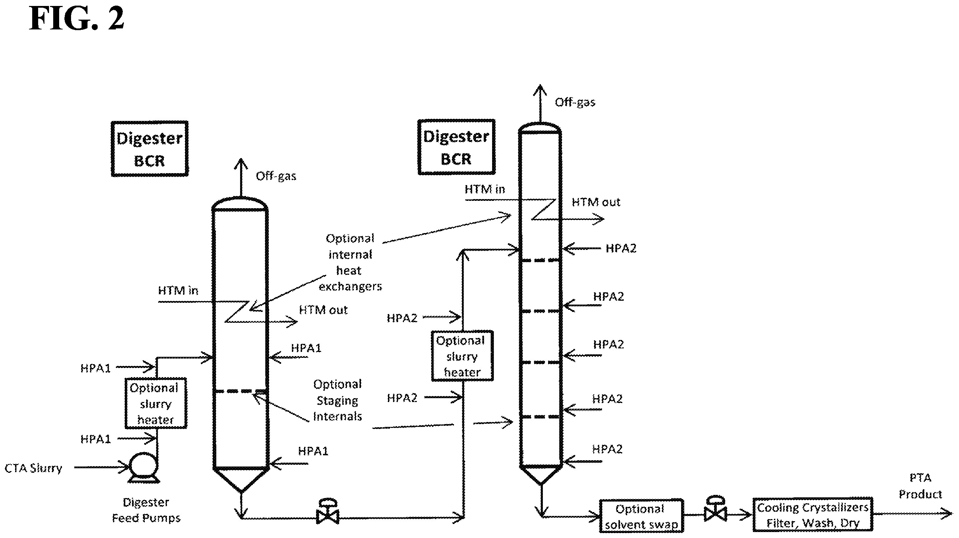

A schematic diagram of a two BCR digestion system according to an embodiment of the present invention is shown in Fig. 2. In this diagram the primary oxidation system as shown in Fig. 1 is not repeated. As in Fig. 1 optional placement of air injection units for the first zone BCR are indicated by HPA1 locations while HPA2 locations are optional placement locations for the air injection units for the second and subsequent BCR digestion zones. In each case one or more of the HPA1 and HPA2 inlets are present in the locations indicated.

As described previously the CTA slurry may be heated in a heat exchange unit prior to entry to the BCR or may be heated within the BCR.

**Claims**

**1.** A method for purification of crude terephthalic acid comprising:

> a) obtaining a digester feed slurry of particles of crude terephthalic acid, comprising terephthalic acid, 4-carboxybenzaldehyde and p-toluic acid in a solvent liquid comprising aqueous acetic acid and a catalyst system comprising at least one heavy metal compound from a primary oxidation reactor;
> b) feeding the crude terephthalic acid slurry to a first digestion zone of a bubble column system in downstream reaction flow from the primary oxidation reactor;
> c) heating the crude terephthalic acid slurry to a temperature of from 150°C to 280°C either before entry to the first digestion zone or when within the first digestion zone;
> d) supplying a gas comprising oxygen to the first digestion zone, wherein the superficial velocity of the gas rising near the top of the first digestion zone is in a range of from 0.1 cm/s to 8 cm/s;
> e) at least partially dissolving particles of crude terephthalic acid in the acetic acid thereby releasing at least some 4-carboxybenzaldehyde and p-toluic acid from the particles and exposing the dissolved 4-carboxybenzaldehyde and p-toluic acid to the oxygen to effect oxidation to terephthalic acid, and to obtain a first stage digester slurry;
> f) passing the first stage digester slurry to a second digestion zone, which is optionally located vertically beneath the first digestion zone;
> g) supplying a gas comprising oxygen to a lower portion of the second digestion zone;
> wherein a supply rate of the gas to the second digestion zone is less than the rate of supply to the first digestion zone;
> h) dissolving and releasing additional 4-carboxybenzaldehyde and p-toluic acid from the particles and exposing the dissolved 4-carboxybenzaldehyde and p-toluic acid to the oxygen to effect additional oxidation to terephthalic acid, and to obtain a second stage digester slurry;
> i) optionally, moving the second stage digester slurry through one or more further digestion zones structured similar to the second digestion zone and optionally vertically beneath the second digestion zone;
> j) removing the resulting terephthalic acid crystal slurry from the last digestion zone; and
> k) isolating the obtained terephthalic acid crystal particles;

> wherein the first digestion zone, the second and optional one or more further digestion zone(s) are free of mechanical agitation.

**2.** The method of claim 1, wherein the superficial velocity of the gas rising in the second digestion zone is less than 1 cm/sec; or:
wherein a mean particle size of the crude terephthalic acid is from 20 to 150 microns; or:

wherein a BET surface area of the crude terephthalic acid is from 0.6 to 4.0 $m^2/g$;
or:
wherein a retention time of the particles in the first digestion zone is from 10 to 60 minutes; or:
wherein a temperature of the CTA slurry within the first digestion zone is from 180 to 230°C; or: wherein a temperature of at least one digestion zone is at least 10°C higher than the temperature of the CTA slurry when obtained from a primary oxidation system; or:
wherein a total residence time of the terephthalic acid particles in the first and second digestion zones is from 60 to 120 minutes; or:
wherein an oxygen content of the exhaust gas is 6% by volume or less, according to dry basis measurement; or:
wherein a mean particle size of the terephthalic acid particle at the outlet of the digestion is from 60 to 100 microns; or:
wherein a gas mixing power summed for all zones of the digestion is less than 0.2 Watt/kg of slurry, preferably wherein the gas mixing power summed for all zones of the digestion is less than 0.05 Watt/kg of slurry; or:
wherein a maximum time-averaged, area averaged bubble hold-up within the bubble column is less than 6 percent; or:
wherein a maximum time-averaged, area averaged bubble hold-up within at least one zone of the bubble column is less than 2 percent; or:
wherein an overall digestion RTD for each of the solid, liquid, and combined slurry phases has a cmF(0.5) of less than 0.35 and a cmF(1.5) of more than 0.80; or: wherein at least 25 percent of the molecular oxygen supply for the first digestion zone is combined with the CTA slurry within 8 minutes after the digester feed slurry is first heated at least 10°C above the temperature at the exit from initial oxidation;
or:
wherein at least 25 percent of the molecular oxygen supply for the first digestion zone is fed into the first digestion zone comingled with the digester feed slurry; or: wherein the temperature of at least 50% of digester feed slurry is increased by at least 10°C using at least one non-contacting heat exchanger apparatus situated external to the bubble column, preferably wherein at least 25 percent of the molecular oxygen supply for the first digestion zone is mixed with the digester feed slurry before the exit of the external heat exchanger.

3. An oxidative digestion system, comprising:

   a series of at least two oxidative digestion zones arranged vertically in one bubble column reactor;
   a reactant inlet located in a lower portion of the first uppermost digestion zone;
   oxygen gas supply inlets to the first uppermost digestion zone and at least one zone in series vertically beneath the first uppermost zone;
   at least one horizontal baffle located between the first uppermost zone and the second zone vertically beneath;
   at least one horizontal baffle located between each respective vertically adjacent zones when more than one zone is present beneath the first uppermost zone;
   a product slurry outlet at the bottom of the at least one bubble column;
   wherein
   the oxidative digestion system is in downstream reaction flow from a primary oxidation reactor and receives a crude reaction product from the primary oxidation reactor;
   the system is free of mechanical agitation;
   each oxygen gas supply comprises a gas distributor unit which feeds the oxygen gas into the zone as a bubbly flow; and
   each horizontal baffle comprises a tray having multiple inverted shaped sloped surfaces with multiple open areas.

4. The oxidation system of claim 3, further comprising an exhaust gas outlet having an oxygen content monitoring system.

5. The oxidation system of claim 3, wherein a total height of the bubble column is from 16 to 40 meters.

6. The oxidation system of claim 3, wherein a diameter of all zones is the same and is from 1.0 meters to 8.0 meters.

7. The oxidation system of claim 3, comprising 3 to 5 zones arranged vertically below the first uppermost zone.

8. The oxidation system of claim 3, wherein a height to diameter ratio of the first uppermost zone is from 1/1 to 4/1.

9. The oxidation system of claim 3, wherein the horizontal baffle comprises a plurality of laterally-spaced baffle members.

**10.** The oxidation system of claim 9, wherein the laterally-spaced baffle members each comprise a substantially cylindrical exposed outer surface.

**11.** The oxidation system of claim 9, wherein the horizontal baffle comprises an inverted V-shaped upwardly-facing exposed outer surface; or:
wherein each horizontal baffle comprises open area of from 25 to 75% of the total horizontal area of the baffle.

**12.** A bubble column digestion system, comprising:

a first BCR unit, structured for convection flow; and
at least one BCR unit structured for plug-flow in series following the first BCR unit;
wherein
the first BCR unit comprises:

a slurry inlet in a central vertical position of the column;
an oxygen containing gas inlet below the slurry inlet;
a slurry outlet at a bottom of the column;
a gas exhaust outlet at a top of the column equipped with an oxygen content monitor; and
optionally, a horizontal baffle between the gas inlet and the slurry outlet; and
wherein
the at least one second BCR unit comprises:

from 1 to 5 horizontally segregated zones, each zone optionally equipped with an oxygen gas inlet;
horizontal baffles between each zone;
a slurry inlet in a highest zone; and
a slurry outlet at a bottom of the BCR unit;
wherein at least one zone is equipped with an oxygen gas inlet;
the oxidative digestion system is in downstream reaction flow from a primary oxidation reactor and receives a crude reaction product from the primary oxidation reactor; and
the system is free of mechanical agitation.


**Patentansprüche**

**1.** Verfahren zur Reinigung von roher Terephthalsäure, bei dem:

a) eine Fermenterzufuhraufschlämmung aus Teilchen roher Terephthalsäure, umfassend Terephthalsäure, 4-Carboxybenzaldehyd und p-Toluolsäure in einer Lösungsmittelflüssigkeit, umfassend wässrige Essigsäure und ein Katalysatorsystem, umfassend mindestens eine Schwermetallverbindung, aus einem primären Oxidationsreaktor erhalten wird;
b) die rohe Terephthalsäureaufschlämmung einer ersten Aufschlusszone eines Blasensäulensystems, der flussabwärts von dem primären Oxidationsreaktor gelegen ist, zugeführt wird;
c) die rohe Terephthalsäureaufschlämmung entweder vor dem Eintritt in die erste Aufschlusszone oder innerhalb der ersten Aufschlusszone auf eine Temperatur von 150 °C bis 280 °C erhitzt wird;
d) ein sauerstoffhaltiges Gas zur ersten Aufschlusszone zugeführt wird, wobei die Oberflächengeschwindigkeit des Gases, das in der Nähe der Oberseite der ersten Aufschlusszone aufsteigt, in einem Bereich von 0,1 cm/s bis 8 cm/s liegt;
e) die Teilchen roher Terephthalsäure zumindest teilweise in der Essigsäure gelöst werden, wodurch mindestens etwas 4-Carboxybenzaldehyd und p-Toluolsäure aus den Teilchen freigesetzt wird und das gelöste 4-Carboxybenzaldehyd und p-Toluolsäure dem Sauerstoff ausgesetzt werden, um eine Oxidation der Terephthalsäure zu bewirken und eine Fermenteraufschlämmung der ersten Stufe zu erhalten;
f) die Fermenteraufschlämmung der ersten Stufe einer zweiten Aufschlusszone, die gegebenenfalls vertikal unterhalb der ersten Aufschlusszone angeordnet ist, zugeführt wird;
g) ein sauerstoffhaltiges Gas einem unteren Teil der zweiten Aufschlusszone zugeführt wird;
wobei eine Zufuhrrate des Gases zu der zweiten Aufschlusszone geringer ist als die Zufuhrrate zu der ersten Aufschlusszone;
h) zusätzliches 4-Carboxybenzaldehyd und p-Toluolsäure aus den Teilchen gelöst und freigesetzt wird und das gelöste 4-Carboxybenzaldehyd und p-Toluolsäure dem Sauerstoff ausgesetzt wird, um eine zusätzliche Oxidation zu Terephthalsäure zu bewirken und eine Fermenteraufschlämmung der zweiten Stufe zu erhalten;

i) die Fermenteraufschlämmung der zweiten Stufe gegebenenfalls durch eine oder mehrere weitere Aufschlusszonen, die ähnlich wie die zweite Aufschlusszone strukturiert sind und gegebenenfalls vertikal unter der zweiten Aufschlusszone angeordnet sind, geführt wird;

j) die resultierende Terephthalsäurekristallaufschlämmung aus der letzten Aufschlusszone entfernt wird; und

k) die erhaltenen Terephthalsäurekristallteilchen isoliert werden;

wobei die erste Aufschlusszone, die zweite und gegebenenfalls eine oder mehrere weitere Aufschlusszonen frei von mechanischer Umwälzung sind.

2. Verfahren nach Anspruch 1, wobei die Oberflächengeschwindigkeit des in der zweiten Aufschlusszone aufsteigenden Gases weniger als 1 cm/s beträgt; oder:

wobei eine mittlere Teilchengröße der rohen Terephthalsäure 20 bis 150 Mikrometer beträgt; oder:

wobei eine BET-Oberfläche der rohen Terephthalsäure 0,6 bis 4,0 m$^2$/g beträgt; oder:

wobei eine Verweilzeit der Teilchen in der ersten Aufschlusszone 10 bis 60 Minuten beträgt; oder:

wobei eine Temperatur der CTA-Aufschlämmung in der ersten Aufschlusszone 180 bis 230 °C beträgt; oder:

wobei eine Temperatur von mindestens einer Aufschlusszone mindestens 10 °C höher ist als die Temperatur der CTA-Aufschlämmung, wenn sie aus einem primären Oxidationssystem erhalten wird; oder:

wobei eine Gesamtverweilzeit der Terephthalsäureteilchen in der ersten und zweiten Aufschlusszone 60 bis 120 Minuten beträgt; oder:

wobei ein Sauerstoffgehalt des Abgases 6 Vol.-% oder weniger gemäß Trockenbasismessung beträgt; oder:

wobei eine mittlere Teilchengröße der Terephthalsäureteilchen am Auslass der Aufschlusszone 60 bis 100 Mikrometer beträgt; oder:

wobei eine für alle Aufschlusszonen summierte Gasmischleistung weniger als 0,2 Watt/kg Aufschlämmung beträgt, vorzugsweise wobei die für alle Aufschlusszonen summierte Gasmischleistung weniger als 0,05 Watt/kg Aufschlämmung beträgt; oder:

wobei ein maximaler zeitgemittelter, flächengemittelter Blasen-Holdup innerhalb der Blasensäule weniger als 6 Prozent beträgt; oder:

wobei ein maximaler zeitgemittelter, flächengemittelter Blasen-Holdup innerhalb mindestens einer Zone der Blasensäule weniger als 2 Prozent beträgt; oder: wobei ein Gesamtaufschluss-RTD für jede der festen, flüssigen und kombinierten Aufschlämmungsphasen einen cmF(0,5) von weniger als 0,35 und einen cmF(1,5) von mehr als 0,80 aufweist; oder:

wobei mindestens 25 Prozent der molekularen Sauerstoffzufuhr für die erste Aufschlusszone innerhalb von 8 Minuten nachdem die Fermenterzufuhraufschlämmung zum ersten mal auf mindestens 10 °C über die Temperatur am Ausgang der anfänglichen Oxidation erhitzt wurde mit der CTA-Aufschlämmung kombiniert werden; oder:

wobei mindestens 25 Prozent der molekularen Sauerstoffzufuhr für die erste Aufschlusszone gemischt mit der Fermenterzufuhraufschlämmung in die erste Aufschlusszone eingespeist werden; oder:

wobei die Temperatur von mindestens 50 % der Fermenterzufuhraufschlämmung unter Verwendung mindestens einer berührungslosen Wärmetauschervorrichtung, die außerhalb der Blasensäule angeordnet ist, um mindestens 10 °C erhöht wird, wobei vorzugsweise mindestens 25 % der molekularen Sauerstoffzufuhr für die erste Aufschlusszone vor dem Austritt aus dem externen Wärmetauscher mit der Fermenterzufuhraufschlämmung gemischt werden.

3. Oxidatives Aufschlusssystem, umfassend:

eine Reihe von mindestens zwei oxidativen Aufschlusszonen, die vertikal in einem Blasensäulenreaktor angeordnet sind;

einen Reaktanteneinlass, der sich in einem unteren Teil der ersten obersten Aufschlusszone befindet;

Sauerstoffgaszufuhreinlässe in die erste oberste Aufschlusszone und mindestens eine Zone in Reihe vertikal unterhalb der ersten obersten Zone;

mindestens eine horizontale Prallplatte, die sich zwischen der ersten obersten Zone und der zweiten vertikal darunter befindlichen Zone;

mindestens eine horizontale Prallplatte, die sich zwischen den jeweiligen vertikal benachbarten Zonen befindet, wenn mehr als eine Zone unterhalb der ersten obersten Zone vorhanden ist;

einen Produktaufschlämmungsauslass am Boden der mindestens einen Blasensäule;

wobei

sich das oxidative Aufschlusssystem flussabwärts von einem primären Oxidationsreaktors befindet und ein Reaktionsrohprodukt von dem primären Oxidationsreaktor erhält;

das System frei von mechanischem Rühren ist;

jede Sauerstoffgasversorgung eine Gasverteilereinheit umfasst, die das Sauerstoffgas als blasenförmigen Strom in die Zone einspeist; und

jede horizontale Prallplatte eine Schale mit mehreren invers geformten, geneigten Oberflächen mit mehreren offenen Bereichen umfasst.

4. Oxidationssystem nach Anspruch 3, ferner umfassend einen Abgasauslass mit einem Sauerstoffgehaltsüberwachungssystem.

5. Oxidationssystem nach Anspruch 3, wobei eine Gesamthöhe der Blasensäule 16 bis 40 Meter beträgt.

6. Oxidationssystem nach Anspruch 3, wobei ein Durchmesser aller Zonen gleich ist und 1,0 m bis 8,0 m beträgt.

7. Oxidationssystem nach Anspruch 3, umfassend 3 bis 5 Zonen, die vertikal unterhalb der ersten obersten Zone angeordnet sind.

8. Oxidationssystem nach Anspruch 3, wobei ein Verhältnis von Höhe zu Durchmesser der ersten obersten Zone 1/1 bis 4/1 beträgt.

9. Oxidationssystem nach Anspruch 3, wobei die horizontale Prallplatte eine Vielzahl von seitlich beabstandeten Prallelementen umfasst.

10. Oxidationssystem nach Anspruch 9, wobei die seitlich beabstandeten Prallelemente jeweils eine freiliegende im Wesentlichen zylindrische Außenfläche umfassen.

11. Oxidationssystem nach Anspruch 9, wobei die horizontale Prallplatte eine inverse V-förmige nach oben gerichtete freiliegende Außenfläche umfasst; oder:

wobei jede horizontale Prallplatte eine offene Fläche von 25 bis 75 % der gesamten horizontalen Fläche der Prallplatte umfasst.

12. Blasensäulenaufschlusssystem, umfassend:

eine erste BCR-Einheit, die für den Konvektionsfluss strukturiert ist; und

mindestens eine BCR-Einheit, die für den Plug-Flow strukturiert ist und in Reihe nach der ersten BCR-Einheit angeordnet ist;

wobei

die erste BCR-Einheit Folgendes umfasst:

einen Aufschlämmungseinlass in einer zentralen vertikalen Position der Säule;

einen Einlass für sauerstoffhaltiges Gas unterhalb des Aufschlämmungseinlasses;

einen Aufschlämmungsauslass am Boden der Säule;

einen Auslass für Abgas an einer Oberseite der Säule, der mit einem Sauerstoffgehaltsmonitor ausgestattet ist; und

gegebenenfalls eine horizontale Prallplatte zwischen dem Gaseinlass und dem Aufschlämmungsauslass; und

wobei

die mindestens eine zweite BCR-Einheit Folgendes umfasst:

1 bis 5 horizontal getrennte Zonen, wobei jede Zone gegebenenfalls mit einem Sauerstoffgaseinlass ausgestattet ist;

horizontale Prallbleche zwischen jeder Zone;

ein Aufschlämmungseinlass in einer höchsten Zone; und

einen Aufschlämmungsauslass an einem Boden der BCR-Einheit;

wobei mindestens eine Zone mit einem Sauerstoffgaseinlass ausgestattet ist;

sich das oxidative Aufschlusssystem flussabwärts von einem primären Oxidationsreaktor befindet und ein Reaktionsrohprodukt von dem primären Oxidationsreaktor erhält; und

das System frei von mechanischem Rühren ist.

**Revendications**

1. Procédé de purification d'acide téréphtalique brut comprenant :

a) obtention d'une suspension d'alimentation de digesteur de particules d'acide téréphtalique brut, comprenant de l'acide téréphtalique, du 4-carboxybenzaldéhyde et de l'acide p-toluique dans un liquide solvant comprenant de l'acide acétique aqueux et un système catalytique comprenant au moins un composé de métal lourd à partir d'un réacteur d'oxydation primaire ;

b) introduction de la suspension d'acide téréphtalique brut dans une première zone de digestion d'un système de colonne à bulles dans un flux réactionnel en aval du réacteur d'oxydation primaire ;

c) chauffage de la suspension d'acide téréphtalique brut à une température comprise entre 150°C et 280°C soit avant l'entrée dans la première zone de digestion soit une fois au sein de la première zone de digestion ;

d) fourniture d'un gaz comprenant de l'oxygène à la première zone de digestion, dans lequel la vitesse superficielle du gaz s'élevant à proximité de la partie supérieure de la première zone de digestion est comprise entre 0,1 cm/s et 8 cm/s ;

e) dissolution au moins partielle des particules d'acide téréphtalique brut dans l'acide acétique libérant ainsi au moins du 4-carboxybenzaldéhyde et de l'acide p-toluique provenant des particules et exposition du 4-carboxybenzaldéhyde et de l'acide p-toluique dissous à l'oxygène afin de réaliser une oxydation en acide téréphtalique, et d'obtenir une suspension de digesteur de premier étage ;

f) passage de la suspension de digesteur de premier étage vers une deuxième zone de digestion, qui est éventuellement située verticalement en-dessous de la première zone de digestion ;

g) fourniture d'un gaz comprenant de l'oxygène à une partie inférieure de la deuxième zone de digestion ; dans lequel un débit d'alimentation du gaz vers la deuxième zone de digestion est inférieur au débit d'alimentation vers la première zone de digestion ;

h) dissolution et libération de 4-carboxybenzaldéhyde et d'acide p-toluique additionnels provenant des particules et exposition du 4-carboxybenzaldéhyde et de l'acide p-toluique dissous à l'oxygène afin de réaliser une oxydation additionnelle en acide téréphtalique, et d'obtenir une suspension de digesteur de deuxième étage ;

i) éventuellement, déplacement de la suspension de digesteur de deuxième étage à travers une ou plusieurs autres zones de digestion structurées de manière similaire à la deuxième zone de digestion et éventuellement verticalement en-dessous de la deuxième zone de digestion ;

j) éliminer la suspension de cristaux d'acide téréphtalique de la dernière zone de digestion ; et

k) isolement des particules de cristaux d'acide téréphtalique obtenues ;

dans lequel la première zone de digestion, la deuxième et éventuellement une ou plusieurs autres zones de digestion sont exemptes d'agitation mécanique.

2. Procédé selon la revendication 1, dans lequel la vitesse superficielle du gaz s'élevant dans la deuxième zone de digestion est inférieure à 1 cm/s ; ou :

dans lequel une taille moyenne de particules de l'acide téréphtalique brut est comprise entre 20 et 150 microns ; ou :

dans lequel une surface BET de l'acide téréphtalique brut est comprise entre 0,6 et 4,0 m$^2$/g ; ou

dans lequel un temps de rétention des particules dans la première zone de digestion est compris entre 10 et 60 minutes ; ou

dans lequel une température de la suspension d'acide téréphtalique brut CTA (Crude Terephtalic Acid) dans la première zone de digestion est comprise entre 180 et 230°C ; ou :

dans lequel une température d'au moins une zone de digestion est supérieure d'au moins 10°C à la température de la suspension de CTA lors de son obtention à partir d'un système d'oxydation primaire ; ou

dans lequel un temps de séjour total des particules d'acide téréphtalique dans les première et deuxième zones de digestion est compris entre 60 et 120 minutes ; ou :

dans lequel une teneur en oxygène du gaz d'échappement est de 6 % en volume ou moins, selon une mesure sur base sèche ; ou

dans lequel une taille moyenne de particules des particules d'acide téréphtalique en sortie de la digestion est comprise entre 60 et 100 microns ; ou

dans lequel une puissance de mélange de gaz additionnée pour toutes les zones de la digestion est inférieure à 0,2 Watt/kg de suspension, préférentiellement dans lequel la puissance de mélange de gaz additionnée pour toutes les zones de la digestion est inférieure à 0,05 Watt/kg de suspension ; ou :

dans lequel une rétention de bulles maximale moyennée dans le temps et sur la zone au sein de la colonne à bulles est inférieure à 6 pour cent ; ou

dans lequel une rétention de bulles maximale moyennée dans le temps et sur la zone au sein d'au moins une zone de la colonne à bulles est inférieure à 2 pour cent ; ou

dans lequel une distribution des temps de séjour RTD (Residence Time Distribution) de digestion globale pour chacune des phases solide, liquide et suspension combinée a une fraction massique cumulative (Cumulative Mass Fraction) cmF(0,5) inférieure à 0,35 et une cmF(1,5) supérieure à 0,80 ; ou :

dans lequel au moins 25 pour cent de l'apport d'oxygène moléculaire pour la première zone de digestion est combinée avec la suspension de CTA dans les 8 minutes qui suivent le premier chauffage de la suspension d'alimentation de digesteur au moins 10°C au-dessus de la température en sortie de l'oxydation initiale ; ou :

dans lequel au moins 25 % de l'apport d'oxygène moléculaire pour la première zone de digestion est introduit dans la première zone de digestion mélangé avec la suspension d'alimentation de digesteur ; ou

dans lequel la température d'au moins 50% de la suspension d'alimentation de digesteur est augmentée d'au moins 10°C en utilisant au moins un appareil échangeur de chaleur sans contact situé à l'extérieur de la colonne à bulles, préférentiellement dans lequel au moins 25 pour cent de l'apport d'oxygène moléculaire pour la première zone de digestion est mélangé à la suspension d'alimentation de digesteur avant la sortie de l'échangeur de chaleur externe.

3.  Système de digestion oxydative, comprenant :

une série d'au moins deux zones de digestion oxydative disposées verticalement dans un réacteur à colonne à bulles ;
une entrée de réactif située dans une partie inférieure de la première zone de digestion la plus haute ;
des entrées d'alimentation en oxygène gazeux vers la première zone de digestion la plus haute et au moins une zone en série verticalement en-dessous de la première zone la plus haute ;
au moins un déflecteur horizontal situé entre la première zone la plus haute et la deuxième zone verticalement en-dessous ;
au moins un déflecteur horizontal situé entre chaque zone respective verticalement adjacente lorsque plus d'une zone est présente en-dessous de la première zone la plus haute ;
une sortie de suspension de produit au niveau de la partie inférieure de l'au moins une colonne à bulles ;
dans lequel
le système de digestion oxydative est dans un flux réactionnel en aval d'un réacteur d'oxydation primaire et reçoit un produit de réaction brut du réacteur d'oxydation primaire ;
le système est exempt d'agitation mécanique ;
chaque alimentation en oxygène gazeux comprend une unité de distribution de gaz qui introduit l'oxygène gazeux dans la zone sous forme d'un flux de bulles ; et
chaque déflecteur horizontal comprend un plateau ayant de multiples surfaces inclinées de forme inversée avec de multiples zones ouvertes.

4.  Système d'oxydation selon la revendication 3, comprenant en outre une sortie de gaz d'échappement ayant un système de surveillance de la teneur en oxygène.

5.  Système d'oxydation selon la revendication 3, dans lequel une hauteur totale de la colonne à bulles est comprise entre 16 et 40 mètres.

6.  Système d'oxydation selon la revendication 3, dans lequel un diamètre de toutes les zones est le même et est compris entre 1,0 mètre et 8,0 mètres.

7.  Système d'oxydation selon la revendication 3, comprenant 3 à 5 zones disposées verticalement en-dessous de la première zone la plus haute.

8.  Système d'oxydation selon la revendication 3, dans lequel un rapport de la hauteur au diamètre de la première zone la plus haute est compris entre 1/1 et 4/1.

9. Système d'oxydation selon la revendication 3, dans lequel le déflecteur horizontal comprend une pluralité d'éléments de déflecteur espacés latéralement.

10. Système d'oxydation selon la revendication 9, dans lequel les éléments de déflecteur espacés latéralement comprennent chacun une surface externe exposée sensiblement cylindrique.

11. Système d'oxydation selon la revendication 9, dans lequel le déflecteur horizontal comprend une surface externe exposée orientée vers le haut en forme de V inversé ;
ou :
dans lequel chaque déflecteur horizontal comprend une surface ouverte de 25 à 75 % de la surface horizontale totale du déflecteur.

12. Système de digestion à colonne à bulles, comprenant :

une première unité de réacteur à colonnes à bulles BCR (Bubble Column Reactor), structurée pour un écoulement par convection ; et
au moins une unité BCR structurée pour un écoulement piston en série après la première unité BCR ;
dans lequel
la première unité BCR comprend :

une entrée de suspension dans une position verticale centrale de la colonne ;
une entrée de gaz contenant de l'oxygène en-dessous de l'entrée de suspension ;
une sortie de suspension au niveau d'une partie inférieure de la colonne ;
une sortie de gaz d'échappement au niveau d'une partie supérieure de la colonne équipée d'un dispositif de surveillance de la teneur en oxygène ; et
éventuellement, un déflecteur horizontal entre l'entrée de gaz et la sortie de suspension ; et
dans lequel
l'au moins une deuxième unité BCR comprend :

de 1 à 5 zones séparées horizontalement, chaque zone étant éventuellement équipée d'une entrée d'oxygène gazeux ;
des déflecteurs horizontaux entre chaque zone ;
une entrée de suspension dans la zone la plus haute ; et
une sortie de suspension au niveau d'une partie inférieure de l'unité BCR ;
dans lequel au moins une zone est équipée d'une entrée d'oxygène gazeux ;
le système de digestion oxydative se trouve dans un flux réactionnel en aval d'un réacteur d'oxydation primaire et reçoit un produit réactionnel brut du réacteur d'oxydation primaire ; et
le système est exempt de toute agitation mécanique.

FIG. 1

FIG. 2

# FIG. 3

Off-gas

Optional
Slurry
Heater

CTA Slurry

Staging Internal

FIG. 4A

FIG. 4B

# FIG. 5A

# FIG. 5B

# FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7355068 B
- US 7371894 B
- US 7568361 B
- US 7829037 B
- US 7910769 B
- US 8501986 B
- US 8685334 B
- US 8790601 B
- US 7393973 B
- CN 202700501
- US 7816556 B2